# EUROPEAN PATENT APPLICATION

(11) **EP 4 796 623 A1**
(43) Date of publication of application: **26.08.2026**
(21) Application number: 25159450.3
(22) Date of filing: 21.02.2025
(51) Int. Cl.: C12C 7/04, C12G 3/021, C12N 1/18

(54) **YEAST STRAINS AND USES THEREOF**

(71) Applicant: DANSTAR FERMENT AG, 6300 Zug (CH)
(72) Inventor: The designation of the inventor has not yet been filed
(74) Representative: J A Kemp LLP

(57) **Abstract**

The invention relates to methods of producing hybrid yeast strains having improved properties for the fermentation of an alcoholic beverage. The invention also relates to methods of producing derivatives of the hybrid yeast strains, and methods for producing a fermented product such as an alcoholic beverage using the yeast strains obtained by the methods of the invention. The invention also relates to the hybrid yeast strains, the derivative yeast strains and a fermentation medium comprising said yeast strains.

## Description

### Field of the invention

The invention relates to methods of producing hybrid yeast strains having improved properties for the fermentation of an alcoholic beverage. The invention also relates to methods of producing derivatives of the hybrid yeast strains, and methods for producing a fermented product such as an alcoholic beverage using the yeast strains obtained by the methods of the invention.

### Background to the invention

Malt is used in the production of alcoholic beverages such as beer and whisky. In the malting process, cereal grains (primarily barley) are steeped in water and the grain is allowed to germinate followed by kilning to produce malted grain. The malted grain is then typically ground and 'mashed': mixed with water and optionally further grains, and heated to allow the amylolytic enzymes in the malt to break down oligosaccharides into less complex sugars such as glucose and maltose which can be readily consumed by yeasts in a subsequent fermentation process. Efficient fermentation requires the rapid and complete utilization of all these sugars. In brewing processes, the wort is typically boiled at the end of the mashing, which inactivates the malt enzymes. In contrast, in whisky production, the wort is not boiled, resulting in the malt enzymes still being active during fermentation and allowing further sugars to fermentable sugars to be accessible to the yeast during fermentation (secondary conversion).

Malt whiskies are made exclusively from malted barley and are distilled in copper pot stills operated in batches. The distillations allow the retention of flavours and obtain complex organoleptic profiles. Grain whiskies are made of malted barley (8-10 %) and other unmodified cereals (90-92 %), typically maize or wheat. Grain whiskies are generally distilled in continuous column stills. In both type of whisky, the malted barley typically supplies the diastatic power during the mashing process: it contains active amylolytic enzymes (α-amylase, β-amylase and limit dextrinase) that hydrolyse starch polysaccharides and oligosaccharides into fermentable sugars. It also provides proteases that hydrolyse proteins into assimilable organic nitrogen, which, together with other nutrients such as vitamins and minerals, is necessary for yeast nutrition during fermentation. As such, grain whisky fermentations typically comprise a lower quantity of amylolytic enzymes during the fermentation step when compared to malt whiskies. According to the Scotch Whisky Regulations 2009, only three ingredients are allowed in Scotch fermentations: water, malted barley (to which only whole grains of other cereals may be added), and yeast. This means that the process relies on the activity of the malt enzymes to liberate fermentable sugars from starch and on the ability of yeast to utilize these fermentable sugars, with maltose and maltotriose being the most abundant (Table 1).

**Table 1: Approximate sugar composition of an all-malt wort from G.M. Walker and A.E. Hill, 2016. Similar figures apply to grain distillery wort.**

| **Sugar** | **Composition** |
|---|---|
| Fructose | 1% |
| Glucose | 10% |
| Maltose | 46% |
| Sucrose | 5% |
| Maltotriose | 15% |
| Maltotetraose | 10% |
| Maltopentaose & higher dextrins | 13% |

Commercial yeasts used for Scotch whisky fermentations have characteristics related to the specific process requirements:
1. Can efficiently ferment the sugars released by malt enzymes (maltose, maltotriose) in addition to simple sugars such as glucose, to ensure cost effective ethanol production.
2. Can tolerate high ethanol concentrations in permissive temperature conditions (generally up to 34 °C).
3. Show fast sugar assimilation kinetics which allow them to compete with the endogenous microflora, including contaminating bacteria and wild yeast. This results in sugar consumption generally completed by 3 to 4 days of fermentation.
4. Contribute to the distinctive flavour profile of Scotch whisky.

One issue faced by most grain distilleries is a limitation in the original gravity of their mash, due to the poor ability of current distilling strains to withstand increased temperatures associated with higher fermentation activity and in the absence of the ability to cool the fermentation vessels (Walker and Hill 2016). Another issue is ensuring that the mashing efficiency is not negatively impacted when the liquor to grist (water to grain) ratio is reduced or mash 'thickness' is increased. By adopting higher original gravities (extract/solids) during mashing both energy demand and water consumption per volume of distillate produced can be reduced.

It is an aim of the present invention to improve fermentation of worts, such as whisk(e)y fermentation processes.

### Summary of the invention

The inventors have surprisingly discovered that the introduction of a functional gene encoding an extracellular glucoamylase into a first yeast strain can result in a new yeast strain having improved properties during an alcoholic fermentation of a whisk(e)y wort, when compared to the first yeast strain, including increased sugar utilisation, increased ethanol production and improved fermentation kinetics, especially when the fermentation is conducted at high temperatures, e.g. at temperatures greater than 34°C. Existing yeast strains used for the fermentation of whisk(e)y may lose performance at high temperatures due to a reduced capacity to utilise maltotriose. Without wishing to be bound by theory, the hybrid yeast strain could increase efficiency of whisk(e)y distilleries by increasing production capacity and by helping to reduce carbon intensity by driving energy savings through optimized resource utilization. This may also reduce efficiency losses incurred during the mashing process, either due to raw material (i.e. malted barley) or process limitations. Furthermore, a strain capable of utilizing previously unfermentable starch degradation components may allow a grain distiller to further reduce the malt inclusion in the process, which would reduce emissions, as malting is both energy and water intensive. The hybrid yeast strain could also be used in other fermentation processes.

Accordingly, the invention relates to a method of obtaining a hybrid yeast strain comprising crossing a first yeast strain with a second yeast strain that comprises a functional gene encoding an extracellular glucoamylase, to thereby produce the hybrid yeast strain, wherein the hybrid yeast strain, when compared to the first yeast strain, is capable of (i) increasing trisaccharide utilisation, (ii)increasing maltose utilisation, (iii) producing more ethanol, (iv) reducing total fermentation time, (v) increasing maximum fermentation rate, and/or (vi) increasing oligosaccharide utilisation, during an alcoholic fermentation of a whisk(e)y wort. The invention also provides a hybrid yeast strain obtained from, or obtainable by, the method of the invention.

The invention also relates to a hybrid yeast strain deposited with the Collection Nationale de Cultures de Microorganismes (CNCM) Patent Depository, on 26 November 2024, with the Deposit No. CNCM I-6150.

The invention also provides a hybrid yeast strain which is a hybrid of a whisk(e)y yeast strain and a yeast strain that comprises a functional gene encoding an extracellular glucoamylase, wherein the hybrid yeast strain is capable of utilising maltotriose, optionally wherein the hybrid yeast strain is a *Saccharomyces cerevisiae x Saccharomyces cerevisiae* var. *diastaticus* hybrid.

The invention additionally relates to a method of producing a derivative yeast strain of the hybrid yeast strain of the invention, comprising crossing the hybrid yeast strain with a further yeast strain to produce a derivative yeast strain which, when compared to an ancestral first yeast strain, is capable of (i) increasing trisaccharide utilisation, (ii)increasing maltose utilisation, (iii) producing more ethanol, (iv) reducing total fermentation time, (v) increasing maximum fermentation rate, and/or (vi) increasing oligosaccharide utilisation, during an alcoholic fermentation of a whisk(e)y wort.

The invention further provides a derivative yeast strain obtained, or obtainable by, the method of producing a derivative yeast strain.

The invention also provides a fermentation medium comprising a whisk(e)y wort and a hybrid or derivative yeast strain according to the invention.

The invention also relates to a method for producing a fermented product, comprising:
(a) optionally performing the method of producing a hybrid yeast strain of the invention;
(b) fermenting a wort or a must with the hybrid yeast strain of (a) or the hybrid or derivative yeast strain according to the invention.

### Brief description of the figures

**Figure 1****.** Ethanol production and residual sugars at 70 h in grain wort S12795 from Scotch fermentations with NT and B24-01: under permissive temperature (20-30°C) and high temperature (18-36°C).
**Figure 2****.** Ankom Fermentation kinetics illustrating cumulative pressure building due to gas release over 70 h fermentations with NT and B24-01 in grain wort S12795 under **A)** permissive temperature (20-30°C) and **B)** high temperature (18-36°C).
**Figure 3****.** PCR analysis of parental strains NT and Belle Saison as well as two colonies isolated from the putative hybrid B23-05. **(A)** Delta PCR analysis. **(B)** PCR analysis targeting a DNA region known to be absent from Belle Saison gDNA. **(C)** PCR analysis targeting STA1 gene from Belle Saison.

### Brief description of the sequence listing

SEQ ID NO: 1 is the coding sequence of the *STA1* gene of strain TUM 71 disclosed in Krogerus *et al* (2019).
SEQ ID NO: 2 is the protein encoded by the *STA1* gene of strain TUM 71 disclosed in Krogerus *et al* (2019). The protein comprises a signal peptide sequence, which may be cleaved co- or post-translationally to produce a protein lacking the signal peptide sequence.
SEQ ID NO: 3 is the promoter sequence of the *STA1* gene of strain TUM 71 disclosed in Krogerus *et al* (2019).
SEQ ID NO: 4 is the sequence of the promoter and the coding sequence of the *STA1* gene of strain TUM 71 disclosed in Krogerus *et al* (2019).
SEQ ID NO: 5 is the sequence of the promoter, coding sequence and terminator sequence of the *STA1* gene of strain TUM 71 disclosed in Krogerus *et al* (2019).

### Detailed description of the invention

The invention relates to the production of new strains of yeast which have improved properties in the alcoholic fermentation of a whisk(e)y wort, when compared to an ancestral yeast. The improved properties including enhanced fermentation kinetics, reduced residual sugars at the end of the fermentation and/or increased ethanol yield. Without wishing to be bound by theory, it is understood that the introduction of a functional gene encoding an extracellular glucoamylase to the ancestral yeast strain is, at least in part, responsible for the improved properties. Glucoamylase expressed extracellularly may be able to hydrolyse oligosaccharides from the malt barley and/or the grains, including maltotriose to glucose and maltose, which are more easily consumed by the yeast from the wort.

The functional gene encoding an extracellular glucoamylase may be introduced by any means, for example by transformation of a plasmid or by CRISPR/Cas techniques. In the method of the disclosure, the functional gene encoding an extracellular glucoamylase is typically introduced by crossing an ancestral yeast strain with a further strain that comprises a functional gene encoding an extracellular glucoamylase.

Accordingly, in a first aspect, the invention relates to a method of obtaining a hybrid yeast strain, wherein the method comprises crossing a first yeast strain with a second yeast strain to thereby produce the hybrid yeast strain. The second yeast strain comprises a functional gene encoding an extracellular glucoamylase. The crossing results in the production of the hybrid yeast strain. The hybrid yeast strain, when compared to the first yeast strain, is capable of (i) increasing trisaccharide utilisation, (ii) increasing maltose utilisation, (iii) producing more ethanol, (iv) reducing total fermentation time, (v) increasing maximum fermentation rate, and/or (vi) increasing oligosaccharide utilisation, during an alcoholic fermentation of a whisk(e)y wort. Typically, the hybrid yeast strain, when also compared to the second yeast strain, is capable of (i) increasing trisaccharide utilisation, (ii) increasing maltose utilisation, (iii) producing more ethanol, (iv) reducing total fermentation time, (v) increasing maximum fermentation rate, and/or (vi) increasing oligosaccharide utilisation, during an alcoholic fermentation of a whisk(e)y wort.

The term *"hybrid yeast strain"* as used herein refers to a strain of yeast that has been produced by the mating of spores from a first yeast strain and a second yeast strain, otherwise referred to herein interchangeably as the crossing or hybridisation of a first yeast strain and a second yeast strain. As discussed in more detail herein, the hybrid yeast strain may be produced directly from the crossing, or one or more further crossing, culturing and/or selection steps. The term *"extracellular glucoamylase"* means an enzyme with glucoamylase activity which, when expressed in a yeast cell, is secreted from the cell. Typically, the extracellular glucoamylase is a soluble glucoamylase. The extracellular glucoamylase may, in some cases, be associated with the cell wall or the membrane of the yeast cell, for example anchored to the cell wall or the membrane of the yeast cell. The term *"comprising a functional gene encoding an extracellular glucoamylase"* may be used interchangeably, for example, with the terms *"expresses an extracellular glucoamylase"* or *"secretes a glucoamylase".*

The terms 'whisky' and 'whiskey' are used interchangeably herein unless explicitly described otherwise. Both terms may be referred to as 'whisky', 'whiskey' or 'whisk(e)y' equally.

### Yeast

The first yeast strain and/or the second yeast strain may be a *Saccharomyces* sp. yeast strain. The first yeast strain and/or the second yeast strain may be a *Saccharomyces cerevisiae* yeast strain. The first yeast strain may be a different species of the *Saccharomyces* genus to the second yeast strain. Typically, the first yeast strain and the second yeast strain are of the same species of the *Saccharomyces* genus. For example, the first yeast strain and the second yeast strain may be strains of *Saccharomyces cerevisiae.* Typically, the first and second yeast strains are different. However, in some cases, the first and second yeast strains may be the same. In such cases, the crossing step involves self-mating of the first and second yeast strain.

The first and second yeast strains are typically not genetically engineered organisms. As used herein, the term *"genetically engineered organism"* refers to an organism comprising novel genetic material introduced through modern biotechnological techniques, such as the transformation of recombinant DNA, CRISPR/Cas techniques or fusion of cells beyond the taxonomic family. Non-genetically engineered organisms include those containing novel genetic material introduced by crossing of cells within the taxonomic family, genus or species, and/or by UV mutagenesis.

The first yeast strain may be a *Saccharomyces* sp. strain. The first yeast strain may be a *Saccharomyces cerevisiae* strain. The first yeast strain may be a yeast strain used for alcoholic fermentation. The first yeast strain is a whisk(e)y yeast strain, such as a whisk(e)y *Saccharomyces cerevisiae* strain. A whisk(e)y yeast strain may be a strain that is used for the fermentation of a whisk(e)y wort. A whisk(e)y yeast strain may be a strain that is used for the fermentation of a grain whisk(e)y wort. A whisk(e)y yeast strain may be a strain that is used for the fermentation of a malt whisk(e)y wort. A whisk(e)y yeast strain may be a strain that is commercially available for the fermentation of the whisk(e)y wort. A whisk(e)y yeast strain is typically capable of efficiently fermenting the sugars released by malt enzymes (such as maltose and maltotriose) and glucose, and can tolerate high ethanol concentrations in permissive temperature conditions, (such as up to 34°C). For example, the first yeast strain may be capable of:
(i) achieving less than 1 % w/v residual glucose, such as less than 0.5 % w/v, 0.1 % w/v or 0.05 % w/v residual glucose;
(ii) achieving less than 2 % w/v residual maltose, such as less than 1% w/v, 0.5 % w/v or 0.4 % w/v residual maltose;
(iii) achieving less than 2 % w/v residual maltotriose, such as less than 1.5 % w/v or less than 1 % w/v residual maltotriose; and/or
(iv) achieving greater than 6 % v/v ethanol, such as greater than 6.5 % v/v, 7 % v/v, 7.5% v/v or 8 % v/v ethanol,
following the fermentation of whisk(e)y wort. The first yeast strain may be capable of achieving all of (i) to (iv) following the fermentation of whisk(e)y wort. The first yeast strain may be capable of achieving less than 2% w/v residual maltotriose and achieving greater than 6% v/v ethanol following the fermentation of a whisk(e)y wort. The first yeast strain may be capable of achieving less than 1.5% w/v residual maltotriose and achieving greater than 6 % v/v ethanol following the fermentation of a whisk(e)y wort. The first yeast strain may be capable of achieving less than 1% w/v residual maltotriose and achieving greater than 6% v/v ethanol following the fermentation of a whisk(e)y wort. The first yeast strain may be capable of achieving less than 1% w/v residual maltotriose and achieving greater than 7% v/v ethanol following the fermentation of a whisk(e)y wort. The first yeast strain may be capable of achieving less than 1% w/v residual maltotriose and achieving greater than 7.5% v/v ethanol following the fermentation of a whisk(e)y wort. The fermentation may be for 72 hours under a temperature gradient of 19°C to 34°C. The whisk(e)y wort may be a malt whisk(e)y wort or a grain whisk(e)y wort.

The first yeast strain may comprise one or more functional genes encoding a maltose permease and/or a maltotriose permease. The maltose permease and the maltotriose permease may be the same protein. As used herein, the terms *"maltose permease"* and *"maltotriose permease"* refer to a protein that transports maltose or maltotriose, respectively, across the yeast cell membrane into the cell. The protein may be an active transporter. The one or more functional genes encoding a maltose permease and/or a maltotriose permease may be from a gene encoding a MAL family transporter. The one or more functional genes encoding a maltose permease and/or a maltotriose permease may be selected from *AGT1, MTY1* and *MAL31* (e.g. as provided in Genbank IDs DQ091763.1 and LT594282.1 and NCBI Ref NM_001178646.1, respectively). The one or more functional genes encoding a maltose permease and/or a maltotriose permease may be selected from *AGT1* and *MTY1.* The first yeast strain may comprise a functional *AGT1* gene. The first yeast strain may comprise a functional *AGT1* and a functional *MTY1* gene. The first yeast strain may comprise a functional *AGT1,* a functional *MTY1* gene and a functional *MAL31* gene. The first yeast strain may comprise two copies of a functional *AGT1* gene. For example, the first yeast strain may comprise two copies of a functional *AGT1* gene, a functional *MTY1* gene and a functional *MAL31 gene.*

A functional *AGT1* gene may comprise a sequence having at least 90% sequence identity with Genbank ID DQ091763.1, such as at least 95%, at least 96%, at least 97%, at least 98%, at least 99% or at least 99.7% sequence identity with Genbank ID DQ091763.1. A functional *MTY1* gene may comprise a sequence having at least 90% sequence identity with Genbank ID LT594282.1, such as at least 95%, or at least 95.5% sequence identity with Genbank ID LT594282.1. A functional *MAL31* gene may comprise a sequence having at least 90% sequence identity with NCBI Ref NM_001178646.1, such as at least 95%, at least 96%, at least 97%, at least 98% or at least 99% sequence identity with NCBI Ref NM_001178646.1.

A functional *AGT1* gene may encode a polypeptide sequence having at least 90% sequence identity with the polypeptide sequence encoded by Genbank ID DQ091763.1, such as at least 95%, at least 96%, at least 97%, at least 98%, at least 99% or at least 99.7% sequence identity with the polypeptide sequence encoded by Genbank ID DQ091763.1. A functional *MTY1* gene may encode a polypeptide sequence having at least 90% sequence identity with the polypeptide sequence encoded by Genbank ID LT594282.1, such as at least 95%, or at least 95.5% sequence identity with the polypeptide sequence encoded by Genbank ID LT594282.1. A functional *MAL31* gene may encode a polypeptide sequence having at least 90% sequence identity with the polypeptide sequence encoded by NCBI Ref NM_001178646.1, such as at least 95%, at least 96%, at least 97%, at least 98% or at least 99% sequence identity with the polypeptide sequence encoded by NCBI Ref NM_001178646.1.

While *S. cerevisiae* strains generally can assimilate maltose, many strains are incapable of fermenting maltotriose (Alves 2008). Maltotriose utilization typically starts after the depletion of other sugars and is highly dependent on the functional expression of permeases such as Agt1 and Mty1 (Day 2022; Dietvorst 2005; Alves 2008). The maltose permease and/or the maltotriose permease may be the maltose permease and/or the maltotriose permease encoded by *AGT1* or *MTY1.* The maltose permease and/or the maltotriose permease may be selected from general alpha-glucoside permease (Uniprot ID P53048), alpha-glucoside permease (Uniprot ID B0M8V5), and MTY1 (GenBank ID SBT28083.1).

Typically, the first yeast strain comprises low or no extracellular glucoamylase activity. A yeast strain comprising low extracellular glucoamylase activity may be a yeast strain comprising the *STA1* gene having a 1162-bp deletion in its promoter, as described in Krogerus *et al* (2019). The 1162-bp deletion is at positions -1370 to -209 from the start codon. The deletion corresponds to positions 1082 to 2243 (inclusive) of any one of SEQ ID NOs: 3 to 5. A yeast strain comprising low extracellular glucoamylase activity may be a yeast strain expressing about the same level of extracellular glucoamylase as a yeast strain comprising the *STA1* gene having a 1162-bp deletion in its promoter, or less than the level of extracellular glucoamylase produced by a yeast strain comprising the *STA1* gene having a 1162-bp deletion in its promoter. The *STA1* gene having a 1162-bp deletion in its promoter may be SEQ ID NO: 4 having a deletion at positions 1082 to 2243 (inclusive), or SEQ ID NO: 5 having a deletion at positions 1082 to 2243 (inclusive). Preferably, the first yeast strain does not comprise a functional gene encoding an extracellular glucoamylase. The first yeast strain preferably does not substantially express an extracellular glucoamylase, e.g. does not express an extracellular glucoamylase at detectable levels. In some cases, the first yeast strain comprises a functional gene encoding a maltose permease and/or maltotriose permease, and does not comprise a functional gene encoding an extracellular glucoamylase.

The first yeast strain may be selected from DistilaMax^{®} NT, DistilaMax^{®} GW, DistilaMax^{®} MW, Pinnacle MG+, Pinnacle^{™}, Pinnacle^{™} MG+, SafSpirits^{™} M1, Kerry M strain, Kerry MX strain and Kerry MDL+.

The second yeast strain may be a *Saccharomyces* sp. strain. The second yeast strain may be a *Saccharomyces cerevisiae* strain. The second yeast strain is typically a diastatic yeast strain. The diastatic yeast strain may be a diastatic *Saccharomyces* sp. strain. The diastatic yeast strain may be a diastatic *Saccharomyces cerevisiae* strain. The second yeast strain may be a *Saccharomyces cerevisiae* var. *diastaticus* strain. The second yeast strain may be selected from DistilaMax^{®} XP, LalBrew Belle Saison^{™} and the diastatic yeasts with a complete STA1 ORF and STA1 promoter described in Krogerus *et al,* 2019 (e.g. TUM PI BA 109, TUM 3-D-2, TUM 71, TUM 3-H-2 and WY3711).

The functional gene encoding an extracellular glucoamylase may be a *STA* gene. The *STA* gene may be *STA1, STA2* or *STA3.* For example, a *STA1* gene comprising a promoter, coding sequence and terminator is provided in SEQ ID NO: 5, a *STA2* gene is provided in Genbank ID M60650.1 and a *STA3* gene is provided in Genbank ID Z71559.1). The functional gene encoding an extracellular glucoamylase is typically *STA1.* Accordingly, the second yeast strain may comprise one or more *STA* genes, such as *STA1, STA2* and/or *STA3.* The second yeast strain may comprise a *STA1* gene. In some cases, the first yeast strain does not comprise a *STA1* gene. In some cases, the first yeast strain does not comprise a *STA* gene.

In some cases, the second yeast strain comprises a functional *STA1* gene. A functional *STA1* gene may comprise a coding sequence having at least 90% sequence identity with the sequence of SEQ ID NO: 1, such as at least 95%, at least 96%, at least 97%, at least 98%, at least 99% or at least 99.7% sequence identity with the sequence of SEQ ID NO: 1. A functional *STA1* gene may encode a polypeptide sequence having at least 90% sequence identity with the sequence of SEQ ID NO: 2, such as at least 95%, at least 96%, at least 97%, at least 98%, at least 99% or at least 99.7% sequence identity with the sequence of SEQ ID NO: 2. A functional *STA1* gene may comprise the full-length promoter sequence of the *STA1* gene as described in Krogerus *et al* (2019), as set out in SEQ ID NO: 3. For example, the *STA1* gene may comprise the upstream activating sequences (UAS) UAS1-2 and UAS2-1 of the *STA1* gene. A functional *STA1* gene may comprise a promoter sequence having at least 90% sequence identity with the sequence of SEQ ID NO: 3 such as at least 95%, at least 96%, at least 97%, at least 98%, at least 99%, at least 99.5%, at least 99.6%, at least 99.7%, at least 99.8%, or at least 99.9% sequence identity with the sequence of SEQ ID NO: 3. A functional *STA1* gene may comprise a sequence having at least 90% sequence identity with the sequence of SEQ ID NO: 4 such as at least 95%, at least 96%, at least 97%, at least 98%, at least 99%, at least 99.5%, at least 99.6%, at least 99.7%, at least 99.8%, or at least 99.9% sequence identity with the sequence of SEQ ID NO: 4. A functional *STA1* gene may comprise a sequence having at least 90% sequence identity with the sequence of SEQ ID NO: 5 such as at least 95%, at least 96%, at least 97%, at least 98%, at least 99%, at least 99.5%, at least 99.6%, at least 99.7%, at least 99.8%, or at least 99.9% sequence identity with the sequence of SEQ ID NO: 5. A functional *STA1* gene may express at least about 50% of the level of extracellular glucoamylase as the *STA1* gene of SEQ ID NO: 4 or 5, when expressed under otherwise identical conditions in *S. cerevisiae,* such as at least about 60%, at least about 70%, at least about 80%, at least about 90% or at least 100% of the level of extracellular glucoamylase as the *STA1* gene of SEQ ID NO: 4 or 5.

The extracellular glucoamylase may be the extracellular glucoamylase encoded by a STA gene, such as STA1. For example, the extracellular glucoamylase may be selected from glucoamylase S1 (SEQ ID NO: 2), glucoamylase S2 (Uniprot ID P29760) and Sta3p (Uniprot ID P87165).

The functional gene encoding the maltose permease and/or the maltotriose permease may be a variant of *AGT1, MTY1* or *MAL31.* The maltose permease and/or the maltotriose permease may be a variant the permease encoded by *AGT1* or *MTY1.* The functional gene encoding an extracellular glucoamylase may be a variant of a *STA* gene, such as *STA1.* The extracellular glucoamylase may be a variant of an extracellular glucoamylase encoded by a *STA* gene, such as *STA1.* As used herein, a variant of a maltose permease retains maltose permease activity, a variant of a maltotriose permease retains maltotriose activity, and a variant of an extracellular glucoamylase retains extracellular glucoamylase activity. The variant may comprise as least 50% identity, such as at least 60% identity, at least 70% identity, at least 80% identity, at least 90% identity, at least 95% identity, at least 98% identity or at least 99% identity with the reference nucleotide or protein sequence. Wherein the reference sequence is a nucleotide sequence, the percentage identity may be calculated against solely the coding sequence of the reference sequence.

The hybrid yeast strain produced by the method of the disclosure may be referred to as a cross of the first and second yeast strains. For example, where the first yeast strain is a *Saccharomyces cerevisiae* strain, e.g. that does not comprise a functional gene encoding an extracellular glucoamylase, and the second yeast strain is a *Saccharomyces cerevisiae* var. *diastaticus* strain, the hybrid strain may be referred to as a *Saccharomyces cerevisiae* x *Saccharomyces cerevisiae* var. *diastaticus* strain.

Without wishing to be limited by theory, it is believed that the method of the disclosure results in a hybrid strain comprising the baseline whisk(e)y fermentation properties of the first yeast strain and further comprises a functional gene encoding the extracellular glucoamylase from the second yeast strain. The hybrid yeast strain may therefore comprise a functional gene encoding an extracellular glucoamylase. The hybrid yeast strain may further comprise a maltose permease and/or a maltotriose permease, such as Agt1, Mty1 or Mal31. Without wishing to be bound by theory, the combination of the glucoamylase and the permeases may be, at least in part, responsible for the improved whisk(e)y fermentation properties of the hybrid yeast strain. It is further believed that the liberation of glucose by the extracellular glucoamylase may influence the expression of genes, such as the permeases, that are repressed by glucose. However, the hybrid yeast strains produced according to the method of the disclosure surprisingly show good improvements over the first and second yeast strains.

*Improved properties during fermentation of a whisk(e)y wort.*

The hybrid yeast strain, when compared to the first yeast strain, is capable of increasing ethanol production, increasing sugar utilisation and improving fermentation kinetics during an alcoholic fermentation of a whisk(e)y wort. Typically, the hybrid yeast strain, when compared to the second yeast strain, is also capable of increasing ethanol production, increasing sugar utilisation and improving fermentation kinetics during an alcoholic fermentation of a whisk(e)y wort.

Sugars can be generally referred to by their degree of polymerisation (DP). Sugars include glucose (a DP1), maltose (a DP2), trisaccharides such as maltotriose (DP3) and oligosaccharides (DP4+, otherwise referred to herein as DP4). The increased sugar utilisation may be increased trisaccharides such as maltotriose utilisation, increased maltose utilisation, and/or increased oligosaccharide utilisation. The increased sugar utilisation may be increased maltotriose utilisation and increased maltose utilisation. The increased sugar utilisation may be increased maltotriose utilisation. Sugar utilisation may be calculated as the residual sugars (% w/v) at the end of fermentation, such as 70 hours after the start of fermentation. Accordingly, the hybrid yeast strain, when compared to the first yeas strain, may be capable of reducing residual sugar levels at the end of an alcoholic fermentation of a whisk(e)y wort, such as at 70 hours after the start of an alcoholic fermentation of a whisk(e)y wort. The reduced residual sugar levels may be reduced trisaccharides such as reduced maltotriose, reduced maltose, and/or reduced oligosaccharide levels. The reduced residual sugar levels may be reduced maltotriose and reduced maltose levels. The reduced residual sugar levels may be reduced maltotriose levels.

The residual maltotriose levels may be reduced by at least about 5%, at least about 10%, at least about 20%, at least about 30% or at least about 40%. The residual maltotriose levels may be reduced by at least about 0.05% w/v, at least about 0.1% w/v, at least about 0.15% w/v, at least about 0.2% w/v, at least about 0.25% w/v, at least about 0.3% w/v or at least about 0.4% w/v. The residual maltotriose levels may be less than about 0.8% w/v, less than about 0.7% w/v, less than about 0.6% w/v, less than about 0.5% w/v, less than about 0.4% w/v or less than about 0.3% w/v.

The residual maltose levels may be reduced by at least about 5%, at least about 10%, at least about 15%, at least about 20% or at least about 25%. The residual maltose levels may be reduced by at least about 0.02% w/v, at least about 0.5% w/v, or at least about 0.1% w/v. The residual maltose levels may be less than about 0.8% w/v, less than about 0.7% w/v, less than about 0.6% w/v, less than about 0.5% w/v, or less than about 0.4% w/v.

The residual oligosaccharide (DP4+) levels may be reduced by at least about 1%, at least about 2%, at least about 3%, at least about 4% or at least about 5%. The residual oligosaccharide (DP4+) levels may be reduced by at least about 0.01% w/v, at least about 0.02% w/v, at least about 0.05% w/v, or at least about 0.1% w/v.

Ethanol production may be increased by at least about 0.5%, at least about 1%, at least about 2%, at least about 3%, at least about 4% or at least about 5%. Ethanol production may be increased by at least about 0.1% v/v, by at least about 0.2% v/v, by at least about 0.3%, by at least about 0.4% or by at least about 0.5%. Ethanol produced by the end of the fermentation, such as at 70 hours after the start of fermentation, may be at least about 8% v/v, at least about 8.5% v/v, or at least about 9% v/v.

The improved fermentation kinetics may be reduced time to fermentation completion, otherwise referred to as "total fermentation time" or "time of fermentation completion". Total fermentation time may be reduced by at least about 1%, at least about 2%, at least about 5% or at least about 10%. Total fermentation time may be reduced by at least about 1 hour, at least about 2 hours, at least about 3 hours, at least about 4 hours, at least about 5 hours, at least about 6 hours, at least about 7 hours, at least about 8 hours, at least about 9 hours or at least about 10 hours. Total fermentation time may be less than about 70 hours, less than about 65 hours, less than about 60 hours, less than about 55 hours or less than about 50 hours. The improved fermentation kinetics may be increased maximum fermentation rate, otherwise referred to as "Vmax". Maximum fermentation rate may be increased by at least about 1%, at least about 2%, at least about 5% or at least about 10%. Maximum fermentation rate may be increased by about 10 mbar/h, by about 20 mbar/h, by about 30 mbar/h, by about 40 mbar/h, by about 50 mbar/h, by about 60 mbar/h or about 70 mbar/h. Maximum fermentation rate may be about 400 mbar/h or more, about 450 mbar/h or more, about 500 mbar/h or more, about 550 bar/h or more, or about 600 mbar/h or more.

Ethanol production, sugar utilisation and fermentation kinetics do not solely depend on the yeast strain, but also varies depending on the wort used and the fermentation conditions used. The skilled person will be able to select suitable combinations of the fermentation properties discussed above based on the data shown in Tables 8 to 11, for example to encompass the values obtained using the S12795 wort substrate. Different properties, e.g. ethanol production, sugar utilisation and fermentation kinetics can be selected independently and combined to arrive at desired properties, such as those encompassing the strains and exemplary fermentations described in the Examples. In these tables, grain wort fermentations were conducted under a permissive temperature ramp of 20°C to 30°C, or in a high temperature ramp of 18°C to 36°C (see Table 14 for details of temperature ramp). The inventors have unexpectedly discovered that the hybrid yeast strains produced according to the disclosure have improved properties especially when fermentation is conducted at a high temperature, as opposed to the permissive temperatures usually used in whisky fermentation. The alcoholic fermentation may be at least partially performed at a temperature of 35°C or greater. The alcoholic fermentation may be at least partially performed at a temperature of 35°C for at least 6 hours, such as least 12 hours, at least about 18 hours, at least about 24 hours, at least about 30 hours or at least about 36 hours. The alcoholic fermentation may be performed using a temperature ramp of 18°C to 36°C, such as following the 18-36 °C ramp provided in Table 14. The alcoholic fermentation may increase from a temperature of 18°C to 36°C over an initial period of 24-36 hours, followed by a temperature of 35°C for at least 6 hours, such as least 12 hours, at least about 18 hours, at least about 24 hours, at least about 30 hours or at least about 36 hours.

The ethanol and/or residual sugar levels are typically measured at the end of fermentation, such as at 70 hours after the start of fermentation. The alcoholic fermentation may be initiated by pitching the wort with about 1.0 gram of dry cell weight per litre.

The hybrid yeast strain of the invention may demonstrate particularly improved properties during the alcoholic fermentation of a grain whisk(e)y wort, due to the lower levels of malt enzymes when compared to a malt whisk(e)y wort. The alcoholic fermentation may be of a malt whisk(e)y wort. The alcoholic fermentation is typically of a grain whisk(e)y wort. The grain whisk(e)y wort may comprise 8-10% malt, wheat grain and backset, and have an original gravity (OG) of 1.065-1.070, a free amino nitrogen content (FAN) of 60-100 ppm and a pH of 4.9 - 5.1.

When compared to the first yeast strain, the hybrid yeast strain may be capable of (i) increasing trisaccharide utilisation, (ii) increasing maltose utilisation, (iii) producing more ethanol, (iv) reducing total fermentation time, (v) increasing maximum fermentation rate, and/or (vi) increasing oligosaccharide utilisation, during an alcoholic fermentation of a whisk(e)y wort. The hybrid strain may be capable of (i) and (ii). The hybrid strain may be capable of (i) and (iii). The hybrid strain may be capable of (i), (ii) and (iii). The hybrid strain may be capable of (i), (ii) and (iv). The hybrid strain may be capable of (i), (iii) and (iv). The hybrid strain may be capable of (i), (ii) (iii) and (iv). The hybrid strain may be capable of (i), (iii), (iv) and (v). The hybrid strain may be capable of (i), (iii), (iv) and (v). The hybrid strain may be capable of (i), (ii), (iii), (iv) and (v). The hybrid strain may be capable of (i), (ii), (iv) and (vi). The hybrid strain may be capable of (i), (iii), (iv) and (vi). The hybrid strain may be capable of (i), (ii) (iii), (iv) and (vi). The hybrid strain may be capable of all of (i) to (iv).

For example, the hybrid yeast strain may be capable of:
- reducing residual maltotriose levels by at least about 5% and reducing residual maltose levels by at least about 5%;
- reducing residual maltotriose levels by at least about 10% and reducing residual maltose levels by at least about 10%;
- reducing residual maltotriose levels by at least about 20% and reducing residual maltose levels by at least about 15%;
- reducing residual maltotriose levels by at least about 30% and reducing residual maltose levels by at least about 20%;
- reducing residual maltotriose levels by at least about 5%, reducing residual maltose levels by at least about 5%, and increasing ethanol production by at least about 0.5%;
- reducing residual maltotriose levels by at least about 10%, reducing residual maltose levels by at least about 10%, and increasing ethanol production by at least about 1%;
- reducing residual maltotriose levels by at least about 20%, reducing residual maltose levels by at least about 15%, and increasing ethanol production by at least about 1.5%; or
- reducing residual maltotriose levels by at least about 30%, reducing residual maltose levels by at least about 20%, and increasing ethanol production by at least about 2%. The hybrid yeast strain, when compared to the first yeast strain, may be capable of maintaining the congener profile of the fermentation product following the fermentation of a whisk(e)y wort. In this context, the term *"maintaining"* means staying within +/- 50% of the levels of the first yeast strain, such as within +/- 40%, within +/- 30%, or within +/- 20% of the levels of the first yeast strain. For the fermentation of a grain whisk(e)y wort, the congener profile may comprise of 1-propanol, isobutanol, 2-methyl-1-butanol, 3-methyl-1-butanol, ethyl acetate, 1-butanol, and ethyl hexanoate. For the fermentation of a malt whisk(e)y wort, the congener profile may further comprise of acetaldehyde, acetal and ethyl butyrate.

### Crossing

The method of the disclosure involves crossing a first yeast strain with a second yeast strain to thereby produce a hybrid yeast strain. The term *"crossing"* means that genetic material, e.g. DNA, from the first yeast strain and the second strain are combined. The term *"crossing"* may be used interchangeably with *"hybridising"* or *"breeding"* herein. Any suitable form of crossing may be used, such as cross-mating, mass-mating and/or self-mating. Cross-mating and mass-mating may be collectively referred to as *"outbreeding".* Cross-mating is the targeted outbreeding of two different yeast strains. Mass-mating involves outbreeding in a population of three or more different yeast strains. Self-mating may be interchangeably referred to herein as *"inbreeding".* Self-mating is the inbreeding of a single yeast strain, e.g. wherein the first yeast strain and the second yeast strain are the same.

Each crossing step typically involves inducing sporulation of the yeast strains and incubating the spores of the first and second yeast strains together. Inducing sporulation may be performed using any method known in the art, for example by contacting the yeast strains with a sporulation medium (1 g/L yeast extract, 10 g/L potassium acetate, 0.5 g/L glucose) for 6 days at 23°C). The method may further comprise preparing the spores with a cell wall digesting agent, such as zymolyase. The incubation step may be performed using any method to allow fusion of spores to produce a hybrid yeast strain, for example in YPD media, incubated at 30°C overnight. For crossing that involves more than one strain, *i.e.* outbreeding, spores of each parental strain may be prepared separately and then combined together. Alternatively, for crossing that involves more than one strain, the spores of the first and second yeast strains may be prepared together. In self-mating, the first and second yeast strains are the same, and so the spores are prepared together. The crossing step typically results in the isolation of a clonal yeast colony (interchangeably referred to herein as a clonal yeast population) corresponding to a single yeast strain, *e.g.* the hybrid yeast strain. The crossing step may comprise outgrowth of a non-clonal population produced by the incubation of spores of the first and second yeast strains together, followed by the isolation of a clonal yeast colony.

The method may comprise cross-mating of a first yeast strain and a second yeast strain to produce a hybrid yeast strain. In other words, the method may comprise outbreeding in a population consisting of the first yeast strain and the second yeast strain. The method may comprise cross-mating of a first yeast strain and a second yeast strain to produce a first hybrid yeast strain, otherwise referred to herein as an intermediate hybrid yeast strain, and one or more further crossing steps. The one or more further crossing steps may comprise (i) self-mating the intermediate hybrid yeast strain, (ii) cross-mating the intermediate hybrid yeast strain with another intermediate hybrid yeast strain, the first yeast strain or the second yeast strain, and/or (iii) mass-mating the intermediate hybrid strain with other intermediate hybrid yeast strain(s), the first yeast strain and/or the second yeast strain. Each of the further crossing steps (i) to (iii) may produce further intermediate hybrid yeast strain(s) for use in additional crossing steps, or may result in the hybrid yeast strain. The method may comprise cross-mating of a first yeast strain and a second yeast strain to produce an intermediate hybrid yeast strain, self-mating of the intermediate hybrid yeast strain to produce a further intermediate hybrid yeast strain, and mass-mating of one or more of the further intermediate hybrid yeast strains and optionally one or more of the intermediate hybrid yeast strains, to thereby produce the hybrid yeast strain.

The method may comprise one or more steps of screening for and selecting a (intermediate) hybrid yeast strain comprising a characteristic of the first yeast strain and of the second yeast strain. The screening step may take place after the first crossing step and optionally after each subsequent crossing step. For example, the characteristic of the first yeast strain may be a genomic sequence (such as the *AGT1* gene) that is present in the first yeast strain but is not present in the second yeast strain. The characteristic of the second yeast strain may be a genomic sequence, such as the functional gene encoding the extracellular glucoamylase (such as the *STA1* gene) that is present in the second yeast strain but is not present in the first yeast strain. In some cases, the first yeast strain and the second yeast strain have resistance to different selective agents. The hybrid yeast strain may comprise resistance to the selective agent from the first yeast strain and resistance to the selective agent of the second yeast strain. In some cases, the method may comprise, prior to the crossing steps, introducing resistance to one or more selective agents in the first yeast strain and/or the second yeast strain, e.g. by generation of spontaneous mutants.

The method may comprise screening for and selecting a (intermediate) hybrid yeast strain, which, when compared to an ancestral first yeast strain, is capable of (i) increasing trisaccharide utilisation, (ii) increasing maltose utilisation, (iii) producing more ethanol, (iv) reducing total fermentation time, (v) increasing maximum fermentation rate, and/or (vi) increasing oligosaccharide utilisation, during an alcoholic fermentation of a whisk(e)y wort. Any method of measuring one or more of (i) to (vi) that is known in the art may be used, for example, the methods used in the Examples.

### Hybrid yeast strains

The disclosure also relates to a hybrid yeast strain obtained from, or obtainable by, a method disclosed herein.

The disclosure also provides the hybrid yeast strain deposited with the Collection Nationale de Cultures de Microorganismes Patent Depository (CNCM), having an address of Institut Pasteur, 25 Rue du Docteur Roux, 75724, Paris, Cedex 15, France, under the ATCC Patent Deposit Designation No. CNCM I-6150 on 26 November 2024. Said strain is also referred to herein as B24-01. The strain has been generated according to the methods set out in the examples and is a *Saccharomyces cerevisiae x Saccharomyces cerevisiae* var. *diastaticus* hybrid.

Also disclosed herein is a hybrid yeast strain which is a hybrid of a whisk(e)y yeast strain and a yeast strain that comprises a functional gene encoding an extracellular glucoamylase, wherein the hybrid yeast strain is capable of utilising maltotriose. The term *"utilising maltotriose"* as used herein refers to the ability of the yeast strain to ferment maltotriose to ethanol and other products including, but not limited to, yeast biomass and congeners.

The hybrid yeast strain may be a *Saccharomyces cerevisiae* x *Saccharomyces cerevisiae* var. *diastaticus* hybrid.

The hybrid yeast strain typically comprises a functional gene encoding an extracellular glucoamylase. The functional gene encoding an extracellular glucoamylase may be a *STA* gene. The *STA* gene may be *STA1, STA2* or *STA3.* The functional gene encoding an extracellular glucoamylase is typically *STA1.* The hybrid yeast strain may comprise one or more *STA* genes, such as *STA1, STA2* and/or *STA3.* The hybrid yeast strain typically comprises a functional *STA1* gene. A functional *STA1* gene may comprise a coding sequence having at least 90% sequence identity with the sequence of SEQ ID NO: 1, such as at least 95%, at least 96%, at least 97%, at least 98%, at least 99% or at least 99.7% sequence identity with the sequence of SEQ ID NO: 1. A functional *STA1* gene may encode a polypeptide sequence having at least 90% sequence identity with the sequence of SEQ ID NO: 2, such as at least 95%, at least 96%, at least 97%, at least 98%, at least 99% or at least 99.7% sequence identity with the sequence of SEQ ID NO: 2. A functional *STA1* gene may comprise the full-length promoter sequence of the *STA1* gene as described in Krogerus *et al* (2019), as set out in SEQ ID NO: 3. For example, the *STA1* gene may comprise the upstream activating sequences (UAS) UAS1-2 and UAS2-1 of the *STA1* gene. A functional *S741* gene may comprise a promoter sequence having at least 90% sequence identity with the sequence of SEQ ID NO: 3 such as at least 95%, at least 96%, at least 97%, at least 98%, at least 99%, at least 99.5%, at least 99.6%, at least 99.7%, at least 99.8%, or at least 99.9% sequence identity with the sequence of SEQ ID NO: 3. A functional *STA1* gene may comprise a sequence having at least 90% sequence identity with the sequence of SEQ ID NO: 4 such as at least 95%, at least 96%, at least 97%, at least 98%, at least 99%, at least 99.5%, at least 99.6%, at least 99.7%, at least 99.8%, or at least 99.9% sequence identity with the sequence of SEQ ID NO: 4. A functional *STA1* gene may comprise a sequence having at least 90% sequence identity with the sequence of SEQ ID NO: 5 such as at least 95%, at least 96%, at least 97%, at least 98%, at least 99%, at least 99.5%, at least 99.6%, at least 99.7%, at least 99.8%, or at least 99.9% sequence identity with the sequence of SEQ ID NO: 5. A functional *STA1* gene may express at least about 50% of the level of extracellular glucoamylase as the *STA1* gene of SEQ ID NO: 4 or 5, when expressed under otherwise identical conditions in *S. cerevisiae,* such as at least about 60%, at least about 70%, at least about 80%, at least about 90% or at least 100% of the level of extracellular glucoamylase as the *STA1* gene of SEQ ID NO: 4 or 5.

The hybrid yeast strain may be capable of:
(a) achieving less than 1 % w/v residual glucose, such as less than 0.5 % w/v, 0.1 % w/v or 0.05 % w/v residual glucose;
(b) achieving less than about 0.8% w/v residual maltose, such as less than about 0.7% w/v, less than about 0.6% w/v, less than about 0.5% w/v, or less than about 0.4% w/v;
(c) achieving less than about 0.8% w/v, such as less than about 0.7% w/v, less than about 0.6% w/v, or less than about 0.5% w/v residual maltotriose; and/or
(d) achieving at least about 8% v/v alcohol, such as at least about 8.5% v/v, or at least about 9% v/v,
following the fermentation of a whisk(e)y wort. The fermentation may be for 72 hours under a temperature gradient of 18-36°C, as described herein. The whisk(e)y wort may be a malt whisk(e)y wort or a grain whisk(e)y wort, as described herein.

The hybrid yeast strain may be capable of:
- achieving less than 0.1% w/v residual glucose, less than 0.5% w/v residual maltose, less than 0.7% w/v maltotriose and at least 8% v/v ethanol;
- achieving less than 0.1% w/v residual glucose, less than 0.5% w/v residual maltose, less than 0.6% w/v maltotriose and at least 8.5% v/v ethanol;
- achieving less than 0.1% w/v residual glucose, less than 0.5% w/v residual maltose, less than 0.5% w/v maltotriose and at least 9% v/v ethanol;
- achieving less than 0.1% w/v residual glucose, less than 0.4% w/v residual maltose, less than 0.5% w/v maltotriose and at least 9% v/v ethanol; or
- achieving less than 0.05% w/v residual glucose, less than 0.4% w/v residual maltose, less than 0.5% w/v maltotriose and at least 9% v/v ethanol;
following the fermentation of a whisk(e)y wort.

The hybrid yeast strain may comprise one or more functional genes encoding a maltose permease and/or a maltotriose permease, e.g. in addition to a functional gene encoding an extracellular glucoamylase. The one or more functional genes encoding a maltose permease and/or a maltotriose permease may be a gene encoding a MAL family transporter. The one or more functional genes encoding a maltose permease and/or a maltotriose permease may be selected from *AGT1, MTY1* and *MAL31* (e.g. as provided in Genbank IDs DQ091763.1 and LT594282.1 and NCBI Ref NM_001178646.1, respectively). The one or more functional genes encoding a maltose permease and/or a maltotriose permease may be selected from *AGT1* and *MTY1.* Thy hybrid yeast strain may comprise a functional *AGT1* gene. The hybrid yeast strain may comprise a functional *AGT1* and a functional *MTY1* gene. The hybrid yeast strain may comprise a functional *AGT1,* a functional *MTY1* gene and a functional *MAL31* gene. A functional *AGT1* gene may comprise a sequence having at least 90% sequence identity with Genbank ID DQ091763.1, such as at least 95%, at least 96%, at least 97%, at least 98%, at least 99% or at least 99.7% sequence identity with Genbank ID DQ091763.1. A functional *MTY1* gene may comprise a sequence having at least 90% sequence identity with Genbank ID LT594282.1, such as at least 95%, or at least 95.5% sequence identity with Genbank ID LT594282.1. A functional *MAL31* gene may comprise a sequence having at least 90% sequence identity with NCBI Ref NM_001178646.1, such as at least 95%, at least 96%, at least 97%, at least 98% or at least 99% sequence identity with NCBI Ref NM_001178646.1. A functional *AGT1* gene may encode a polypeptide sequence having at least 90% sequence identity with the polypeptide sequence encoded by Genbank ID DQ091763.1, such as at least 95%, at least 96%, at least 97%, at least 98%, at least 99% or at least 99.7% sequence identity with the polypeptide sequence encoded by Genbank ID DQ091763.1. A functional *MTY1* gene may encode a polypeptide sequence having at least 90% sequence identity with the polypeptide sequence encoded by Genbank ID LT594282.1, such as at least 95%, or at least 95.5% sequence identity with the polypeptide sequence encoded by Genbank ID LT594282.1. A functional *MAL31* gene may encode a polypeptide sequence having at least 90% sequence identity with the polypeptide sequence encoded by NCBI Ref NM_001178646.1, such as at least 95%, at least 96%, at least 97%, at least 98% or at least 99% sequence identity with the polypeptide sequence encoded by NCBI Ref NM_001178646.1.

The hybrid yeast strain may be capable of, when compared to an ancestral yeast strain, (i) increasing trisaccharide utilisation, (ii) increasing maltose utilisation, (iii) producing more ethanol, (iv) reducing total fermentation time, (v) increasing maximum fermentation rate, and/or (vi) increasing oligosaccharide utilisation, during an alcoholic fermentation of a whisk(e)y wort. The ancestral yeast strain may be the first yeast strain from which the hybrid yeast strain is derived. The first yeast strain may be as described herein. The conditions of fermentation may be as described herein for the method of producing a hybrid yeast strain. For example, the alcoholic fermentation may be performed using a temperature ramp of 18°C to 36°C over an initial period of 24-36 hours, followed by a temperature of at least 35°C for at least about 36 hours, wherein the alcoholic fermentation is initiated by pitching the wort with about 1.0 gram of dry cell weight per litre, and wherein the wort is a grain whisk(e)y wort comprising 8-10% malt, wheat grain and backset, and have an original gravity (OG) of 1.065-1.070 , a free amino nitrogen content (FAN) of 60-100 ppm and a pH of 4.9 - 5.1.

The hybrid yeast strain may be also capable of, when compared to a further ancestral yeast strain, (i) increasing trisaccharide utilisation, (ii) increasing maltose utilisation, (iii) producing more ethanol, (iv) reducing total fermentation time, (v) increasing maximum fermentation rate, and/or (vi) increasing oligosaccharide utilisation, during an alcoholic fermentation of a whisk(e)y wort. The further ancestral yeast strain may be the second yeast strain from which the hybrid strain is derived. The second yeast strain may be as described herein.

Each of (i) to (vi) may be as described herein.

Accordingly, the disclosure also provides a hybrid yeast strain that is capable of:
(a) achieving less than 1 % w/v residual glucose, such as less than 0.5 % w/v, 0.1 % w/v or 0.05 % w/v residual glucose;
(b) achieving less than about 0.8% w/v residual maltose, such as less than about 0.7% w/v, less than about 0.6% w/v, less than about 0.5% w/v, or less than about 0.4% w/v;
(c) achieving less than about 0.8% w/v, such as less than about 0.7% w/v, less than about 0.6% w/v, less than about 0.5% w/v, less than about 0.4% w/v or less than about 0.3% w/v residual maltotriose; and/or
(d) achieving at least about 8% v/v alcohol, such as at least about 8.5% v/v, or at least about 9% v/v,
following the fermentation of whisk(e)y wort. The fermentation may be for 72 hours under a temperature gradient of 18-36 °C. The whisk(e)y wort may be as described herein.

The hybrid yeast strain may be capable of:
- achieving less than 0.1% w/v residual glucose, less than 0.5% w/v residual maltose, less than 0.7% w/v maltotriose and at least 8% v/v ethanol;
- achieving less than 0.1% w/v residual glucose, less than 0.5% w/v residual maltose, less than 0.6% w/v maltotriose and at least 8.5% v/v ethanol;
- achieving less than 0.1% w/v residual glucose, less than 0.5% w/v residual maltose, less than 0.5% w/v maltotriose and at least 9% v/v ethanol;
- achieving less than 0.1% w/v residual glucose, less than 0.4% w/v residual maltose, less than 0.5% w/v maltotriose and at least 9% v/v ethanol; or
- achieving less than 0.05% w/v residual glucose, less than 0.4% w/v residual maltose, less than 0.5% w/v maltotriose and at least 9% v/v ethanol;
following the fermentation of whisk(e)y wort.

The hybrid yeast strain comprises a functional gene encoding an extracellular glucoamylase. The functional gene encoding an extracellular glucoamylase may be a *STA* gene. The *STA* gene may be *STA1, STA2* or *STA3.* The functional gene encoding an extracellular glucoamylase is typically *STA1.* The hybrid yeast strain may comprise one or more *STA* genes, such as *STA1, STA2* and/or *STA3.* The hybrid yeast strain comprises one or more functional genes encoding a maltose permease and/or a maltotriose permease, such as *AGT1, MTY1* and/or *MAL31.* The hybrid yeast strain may comprise, for example, a functional *STA1* gene, and a functional *AGT1* and/or *MTY1* gene. The hybrid yeast strain may comprise, for example, a functional *STA1* gene, and a functional *AGT1, MTY1* and/or *MAL31* gene.

The ancestral yeast strain of the hybrid yeast strain may be identified by any means known to the skilled person. The ancestral yeast strain may have the properties of the first yeast strain as described herein. For example, strain fingerprinting by Delta-PCR using primers targeting inter-delta sequences may be performed between the hybrid yeast strain and the ancestral *Saccharomyces cerevisiae* yeast strain to confirm an ancestral relationship. Delta-PCR analysis is described in Legras, J. L., & Karst, F. (2003). Optimisation of interdelta analysis for Saccharomyces cerevisiae strain characterisation. FEMS microbiology letters, 221(2), 249-255, and Xufre et al (2011). Use of interdelta polymorphisms of Saccharomyces cerevisiae strains to monitor population evolution during wine fermentation. Journal of Industrial Microbiology and Biotechnology, 38(1), 127-132.

### Derivatives

The disclosure also provides derivatives of the hybrid yeast strains disclosed herein. Accordingly, provided herein is a method of producing a derivative yeast strain of the hybrid yeast strain as described herein. The method comprises crossing the hybrid yeast strain with a further yeast strain to produce a derivative yeast strain. Further provided herein is a method of producing a derivative yeast strain, wherein the method comprises crossing the yeast strain deposited with the Collection Nationale de Cultures de Microorganismes Patent Depository, having an address of Institut Pasteur, 25 Rue du Docteur Roux, 75724, Paris, Cedex 15, France, under the ATCC Patent Deposit Designation No. CNCM I-6150 on 26 November 2024. Said strain is also referred to herein as B24-01. The strain has been generated according to the methods set out in the examples and is a *Saccharomyces cerevisiae* x *Saccharomyces cerevisiae* var. *diastaticus* hybrid.

The derivative yeast strain typically comprises a functional gene encoding an extracellular glucoamylase. The functional gene encoding an extracellular glucoamylase may be a *STA* gene. The *STA* gene may be *STA1, STA2* or *STA3.* The functional gene encoding an extracellular glucoamylase is typically *STA1.* The derivative yeast strain may comprise one or more *STA* genes, such as *STA1, STA2* and/or *STA3.* The derivative yeast strain typically comprises a functional *STA1* gene. A functional *STA1* gene may comprise a coding sequence having at least 90% sequence identity with the sequence of SEQ ID NO: 1, such as at least 95%, at least 96%, at least 97%, at least 98%, at least 99% or at least 99.7% sequence identity with the sequence of SEQ ID NO: 1. A functional *STA1* gene may encode a polypeptide sequence having at least 90% sequence identity with the sequence of SEQ ID NO: 2, such as at least 95%, at least 96%, at least 97%, at least 98%, at least 99% or at least 99.7% sequence identity with the sequence of SEQ ID NO: 2. A functional *STA1* gene may comprise the full-length promoter sequence of the *STA1* gene as described in Krogerus *et al* (2019), as set out in SEQ ID NO: 3. For example, the *STA1* gene may comprise the upstream activating sequences (UAS) UAS1-2 and UAS2-1 of the *STA1* gene. A functional *STA1* gene may comprise a promoter sequence having at least 90% sequence identity with the sequence of SEQ ID NO: 3 such as at least 95%, at least 96%, at least 97%, at least 98%, at least 99%, at least 99.5%, at least 99.6%, at least 99.7%, at least 99.8%, or at least 99.9% sequence identity with the sequence of SEQ ID NO: 3. A functional *STA1* gene may comprise a sequence having at least 90% sequence identity with the sequence of SEQ ID NO: 4 such as at least 95%, at least 96%, at least 97%, at least 98%, at least 99%, at least 99.5%, at least 99.6%, at least 99.7%, at least 99.8%, or at least 99.9% sequence identity with the sequence of SEQ ID NO: 4. A functional *STA1* gene may comprise a sequence having at least 90% sequence identity with the sequence of SEQ ID NO: 5 such as at least 95%, at least 96%, at least 97%, at least 98%, at least 99%, at least 99.5%, at least 99.6%, at least 99.7%, at least 99.8%, or at least 99.9% sequence identity with the sequence of SEQ ID NO: 5. A functional *STA1* gene may express at least about 50% of the level of extracellular glucoamylase as the *STA1* gene of SEQ ID NO: 4 or 5, when expressed under otherwise identical conditions in *S. cerevisiae,* such as at least about 60%, at least about 70%, at least about 80%, at least about 90% or at least 100% of the level of extracellular glucoamylase as the *STA1* gene of SEQ ID NO: 4 or 5.

The derivative yeast strain may be capable of:
(a) achieving less than 1 % w/v residual glucose, such as less than 0.5 % w/v, 0.1 % w/v or 0.05 % w/v residual glucose;
(b) achieving less than about 0.8% w/v residual maltose, such as less than about 0.7% w/v, less than about 0.6% w/v, less than about 0.5% w/v, or less than about 0.4% w/v;
(c) achieving less than about 0.8% w/v, such as less than about 0.7% w/v, less than about 0.6% w/v, less than about 0.5% w/v, less than about 0.4% w/v or less than about 0.3% w/v residual maltotriose; and/or
(d) achieving at least about 8% v/v alcohol, such as at least about 8.5% v/v, or at least about 9% v/v, following the fermentation of whisk(e)y wort. The conditions of fermentation may be as described herein for the method of producing a hybrid yeast strain. For example, the alcoholic fermentation may be performed using a temperature ramp of 18°C to 36°C over an initial period of 24-36 hours, followed by a temperature of at least 35°C for at least about 36 hours, wherein the alcoholic fermentation is initiated by pitching the wort with about 1.0 gram of dry cell weight per litre, and wherein the wort is a grain whisk(e)y wort comprising 8-10% malt, wheat grain and backset, and have an original gravity (OG) of 1.065-1.070, a free amino nitrogen content (FAN) of 60-100 ppm and a pH of 4.9 to 5.1.

The derivative yeast strain may be capable of:
- achieving less than 0.1% w/v residual glucose, less than 0.5% w/v residual maltose, less than 0.7% w/v maltotriose and at least 8% v/v ethanol;
- achieving less than 0.1% w/v residual glucose, less than 0.5% w/v residual maltose, less than 0.6% w/v maltotriose and at least 8.5% v/v ethanol;
- achieving less than 0.1% w/v residual glucose, less than 0.5% w/v residual maltose, less than 0.5% w/v maltotriose and at least 9% v/v ethanol;
- achieving less than 0.1% w/v residual glucose, less than 0.4% w/v residual maltose, less than 0.5% w/v maltotriose and at least 9% v/v ethanol; or
- achieving less than 0.05% w/v residual glucose, less than 0.4% w/v residual maltose, less than 0.5% w/v maltotriose and at least 9% v/v ethanol;
following the fermentation of whisk(e)y wort.

The derivative yeast strain may comprise one or more functional genes encoding a maltose permease and/or a maltotriose permease, e.g. in addition to a functional gene encoding an extracellular glucoamylase. The one or more functional genes encoding a maltose permease and/or a maltotriose permease may be a gene encoding a MAL family transporter. The one or more functional genes encoding a maltose permease and/or a maltotriose permease may be selected from *AGT1, MTY1* and *MAL31* (e.g. as provided in Genbank IDs DQ091763.1 and LT594282.1 and NCBI Ref NM_001178646.1, respectively). The one or more functional genes encoding a maltose permease and/or a maltotriose permease may be selected from *AGT1* and/or *MTY1.* The derivative yeast strain may comprise, for example, a functional *STA1* gene, and a functional *AGT1* and/or *MTY1* gene. Thy hybrid yeast strain may comprise a functional *AGT1* gene. The hybrid yeast strain may comprise a functional *AGT1* and a functional *MTY1* gene. The hybrid yeast strain may comprise a functional *AGT1,* a functional *MTY1* gene and a functional *MAL31* gene. A functional *AGT1* gene may comprise a sequence having at least 90% sequence identity with Genbank ID DQ091763.1, such as at least 95%, at least 96%, at least 97%, at least 98%, at least 99% or at least 99.7% sequence identity with Genbank ID DQ091763.1. A functional *MTY1* gene may comprise a sequence having at least 90% sequence identity with Genbank ID LT594282.1, such as at least 95%, or at least 95.5% sequence identity with Genbank ID LT594282.1. A functional *MAL31* gene may comprise a sequence having at least 90% sequence identity with NCBI Ref NM_001178646.1, such as at least 95%, at least 96%, at least 97%, at least 98% or at least 99% sequence identity with NCBI Ref NM_001178646.1. A functional *AGT1* gene may encode a polypeptide sequence having at least 90% sequence identity with the polypeptide sequence encoded by Genbank ID DQ091763.1, such as at least 95%, at least 96%, at least 97%, at least 98%, at least 99% or at least 99.7% sequence identity with the polypeptide sequence encoded by Genbank ID DQ091763.1. A functional *MTY1* gene may encode a polypeptide sequence having at least 90% sequence identity with the polypeptide sequence encoded by Genbank ID LT594282.1, such as at least 95%, or at least 95.5% sequence identity with the polypeptide sequence encoded by Genbank ID LT594282.1. A functional *MAL31* gene may encode a polypeptide sequence having at least 90% sequence identity with the polypeptide sequence encoded by NCBI Ref NM_001178646.1, such as at least 95%, at least 96%, at least 97%, at least 98% or at least 99% sequence identity with the polypeptide sequence encoded by NCBI Ref NM_001178646.1.

When compared to an ancestral yeast strain, the derivative yeast strain may be capable of (i) increasing trisaccharide utilisation, (ii) increasing maltose utilisation, (iii) producing more ethanol, (iv) reducing total fermentation time, (v) increasing maximum fermentation rate, and/or (vi) increasing oligosaccharide utilisation, during an alcoholic fermentation of a whisk(e)y wort. The ancestral yeast strain may be the first yeast strain from which the hybrid yeast strain is derived. The first yeast strain may be as described herein.

The derivative yeast strain is typically also capable of, when compared to a further ancestral yeast strain, (i) increasing trisaccharide utilisation, (ii) increasing maltose utilisation, (iii) producing more ethanol, (iv) reducing total fermentation time, (v) increasing maximum fermentation rate, and/or (vi) increasing oligosaccharide utilisation, during an alcoholic fermentation of a whisk(e)y wort. The further ancestral yeast strain may be the second yeast strain from which the hybrid strain is derived. The second yeast strain may be as described herein.

Each of (i) to (vi) may be as described herein.

The derivative yeast strain may be identified as a derivative of the hybrid yeast strain by any means known to the skilled person. The derivative yeast strain comprises the properties of the hybrid yeast strain as described herein. Delta-PCR analysis may be performed between the hybrid yeast strain and the derivative yeast strain to confirm an ancestral relationship. Delta-PCR analysis is described in Legras, J. L., & Karst, F. (2003). Optimisation of interdelta analysis for Saccharomyces cerevisiae strain characterisation. FEMS microbiology letters, 221(2), 249-255, and Xufre et al (2011). Use of interdelta polymorphisms of Saccharomyces cerevisiae strains to monitor population evolution during wine fermentation. Journal of Industrial Microbiology and Biotechnology, 38(1), 127-132.

The further yeast strain may be any yeast strain. The further yeast strain may be of the same genus as the hybrid yeast strain. The further yeast strain is typically of the same species as the hybrid yeast strain. The further yeast strain may be a hybrid yeast strain as described herein. The further yeast strain may be the hybrid yeast strain. The further yeast species may be a *Saccharomyces* sp. yeast strain. The further yeast strain may be a *Saccharomyces* cerevisiae yeast strain. The further yeast strain may be a *Saccharomyces cerevisiae* var. *diastaticus* strain. The further yeast strain may be *Saccharomyces cerevisiae* x *Saccharomyces* cerevisiae var. *diastaticus* hybrid strain. The further yeast strain is typically not a genetically engineered organism.

The method may further comprise another step of crossing the derivative yeast strain with a further yeast strain, as described herein, to produce a further derivative yeast strain. The further derivative yeast strain typically comprises the properties described above for the derivative yeast strain. The method may be repeated one or more times. There is no theoretical limit to the number of crossing steps performed in the method, provided the properties of the derivative yeast strain described herein are maintained.

Any suitable form of crossing may be used, such as cross-mating, mass-mating and/or self-mating, as described herein. The method may comprise cross-mating and mass-mating. The method may cross-mating and self-mating. The method may comprise mass-mating and self-mating. The method may comprise cross-mating, mass-mating and self-mating.

The method may comprise screening for and selecting a derivative yeast strain comprising one or more characteristics of the hybrid yeast strain. The characteristics of the hybrid yeast strain may be any of those described herein.

Also provided is a derivative yeast strain that is obtained, or obtainable by the method of producing a derivative yeast strain as described herein. The derivative yeast strain may have the functional and/or structural properties as described herein.

### Fermentation medium

Also provided herein is a fermentation medium comprising a whisk(e)y wort and a hybrid or derivative yeast strain as described herein. As discussed, the hybrid and derivative yeast strains have improved properties during the fermentation of a whisk(e)y wort, when compared to an ancestral yeast strain. The whisk(e)y wort may be a grain whisk(e)y wort or a malt whisk(e)y wort.

The whisk(e)y wort may be a grain whisk(e)y wort. As described herein, the hybrid or derivative yeast strains are typically capable of increasing maltotriose, maltose and/or oligosaccharide utilisation. This may allow lower levels of malt to be included in the wort due to the lower need for malt enzymes to produce similar levels of sugars available to the yeast. The grain whisk(e)y wort may comprise malt, grain and optionally backset. The grain whisk(e)y wort may comprise about 5 to about 15% (w/w) malt, such as about 5 to about 12% malt, about 5 to about 11% malt, about 5 to about 10% malt, about 5 to about 9% malt, about 5 to about 8% malt or about 6 to about 8% malt. The grain whisk(e)y wort may comprise about 5%, about 6%, about 7%, about 8%, about 9%, about 10%, about 11% malt, or about

12% malt. Grain whisk(e)y worts typically comprise about 8% to about 11% malt. The grain may be wheat and/or maize. The grain whisk(e)y wort may comprise up to about 50% backset, such as up to about 45% backset, up to about 40% backset, up to about 30% backset, up to about 20% backset or up to about 10% backset. The grain whisk(e)y wort may comprise about 1% to about 50% backset, such as about 5% to about 45% backset or about 5% to about 20% backset. The grain whisk(e)y wort may have an original gravity (OG) of about 1.050 to about 1.090, such as about 1.060 to about 1.080. The grain whisk(e)y wort may have an original gravity (OG) of about 1.065 to about 1.070, or 1.065 to 1.070 (the standard gravity of a grain whisk(e)y wort). The grain whisk(e)y wort may have an original gravity (OG) of about 1.070 to about 1.090, such as 1.070 to 1.090 (high gravity for a whisk(e)y wort). The grain whisk(e)y wort may comprise about 50 ppm to about 500 ppm of free amino nitrogen (FAN), such as about 50 ppm to 250 ppm of FAN. The grain whisk(e)y wort may have a pH of about 4.5 to about 7, such as about 4.5 to about 6.5, about 4.5 to about 6.0, about 4.6 to about 5.5, or about 4.7 to about 5.4.

The whisk(e)y wort may be a malt whisk(e)y wort. The malt whisk(e)y wort may have an original gravity (OG) of about 1.050 to about 1.090, such as about 1.060 to about 1.080. The malt whisk(e)y wort may have an original gravity (OG) of about 1.065 to about 1.070, or 1.065 to 1.070 (the standard gravity of a malt whisk(e)y wort). The malt whisk(e)y wort may have an original gravity (OG) of about 1.070 to about 1.090, such as 1.070 to 1.090 (high gravity for a whisk(e)y wort). The malt whisk(e)y wort may comprise about 50 ppm to about 1000 ppm of free amino nitrogen (FAN), such as about 100 ppm to 1000 ppm of FAN. The grain whisk(e)y wort may have a pH of about 4.5 to about 7, such as about 4.5 to about 6.0, about 4.6 to about 6.0, or about 4.67 to about 5.92.

The whisk(e)y wort may comprise about 0.1 to about 10 grams of hybrid or derivative yeast cell (dry cell weight per litre), such as about 0.5 to about 5 grams of dry cell weight per litre or about 1.0 to about 2.0 grams of hybrid or derivative yeast cell (dry cell weight per litre). The fermentation medium may consist of the whisk(e)y wort as described herein and the hybrid or derivative yeast strain as described herein, *i.e.* does not comprise any other ingredients.

### Method for producing a fermented product

Also provided is a method for producing a fermented product. The method comprises fermenting a wort or a must with a hybrid yeast strain or derivative yeast strain as described herein.

The method may comprise performing the method of producing a hybrid yeast strain as described herein and/or performing the method of producing a derivative yeast strain as described herein. Accordingly, the method may comprise (a) performing the method of producing a hybrid yeast strain as described herein and/or performing the method of producing a derivative yeast strain as described herein, to thereby produce a hybrid yeast strain or a derivative yeast strain, and (b) fermenting a wort or a must with the hybrid yeast strain or the derivative yeast strain produced in step (a). The fermented product is typically a fermented beverage.

As described herein, the hybrid and derivative yeast strains of the disclosure have improved properties during the fermentation of an alcoholic wort. The improved properties may result from the expression of an extracellular glucoamylase by the hybrid or derivative yeast strains. Diastatic yeasts express an extracellular glucoamylase and may be used in the beer brewing industry. Accordingly, in one aspect, the wort is a beer wort. In such aspects, the fermented product in a beer. In a preferred aspect, the wort is a whisk(e)y wort, such as a grain whisk(e)y wort or a malt whisk(e)y wort. In this aspect, the fermented product is a whisk(e)y. The whisk(e)y wort or the malt whisk(e)y wort may be as further described herein above. The fermented product may a Scotch whisky as defined in the 2009 Scotch Whisky Regulations. The fermented product may a blended Scotch whisky as defined in the 2009 Scotch Whisky Regulations. The fermented product may a single malt Scotch whisky as defined in the 2009 Scotch Whisky Regulations.

The method may comprise a further step of distilling the product produced in the fermentation step. The method may comprise a further step of maturing the product produced in the fermentation step. The method may comprise distilling the product produced in the fermentation step, and maturing the distillation product, to thereby produce the fermented product.

The fermentation step may be at least partially performed at a temperature of 35°C or greater. The alcoholic fermentation may be at least partially performed at a temperature of 35°C for at least 6 hours, such as least 12 hours, at least about 18 hours, at least about 24 hours, at least about 30 hours or at least about 36 hours. The alcoholic fermentation may be performed using a temperature ramp of 18°C to 36°C. The alcoholic fermentation may increase from a temperature of 18°C to 36°C over an initial period of 24-36 hours, followed by a temperature of 35°C for at least 6 hours, such as least 12 hours, at least about 18 hours, at least about 24 hours, at least about 30 hours or at least about 36 hours. The total time of fermentation may be about 40 to about 96 hours, such as about 40 to about 72 hours, about 40 to about 64 hours, or about 44 to about 60 hours. As described herein, the hybrid or derivative yeast strains of the disclosure may have improved fermentation properties at high temperatures (35°C or greater) and reduced times of fermentation completion, relative to existing yeast strains.

The alcoholic fermentation may be initiated by pitching the wort with about 0.1 to about 10 grams of dry cell weight per litre, such as about 0.5 to about 5 grams of dry cell weight per litre or about 1.0 to about 2.0 grams of dry cell weight per litre.

The fermentation may be a batch fermentation. The fermentation may be a continuous fermentation.

### Further definitions

Unless defined otherwise, technical and scientific terms used herein have the same meaning as commonly understood by a person skilled in the art to which this invention belongs.

In general, the term *"comprising"* is intended to mean including but not limited to. For example, the phrase *"a method comprising crossing two yeast strains"* should be interpreted to mean that the method comprises a step of crossing the two yeast strains, but may comprise further steps (for example selection steps and further crossing steps involving other yeast strains).

In some embodiments of the invention, the word *"comprising"* is replaced with the phrase *"consisting of".* The term *"consisting of"* is intended to be limiting. For example, the phrase *"a wort consisting essentially of malt, grain and optionally backset"* should be understood to mean that the wort contains malt, grain and optionally backset, and no other ingredients, such as exogenously added glucoamylase enzyme.

In some embodiments of the invention, the word *"comprising"* is replaced with the phrase *"consisting essentially of".* The term *"consisting essentially of"* means that specific further components can be present, namely those not materially affecting the essential characteristics of the subject matter. For example, the phrase *"a wort consisting essentially of malt, grain and optionally backset"* indicates that the medium may comprise one or more contaminants that have no particular function.

For the purpose of this invention, in order to determine the percent identity of two sequences (such as two polynucleotide or two polypeptide sequences), the sequences are aligned for optimal comparison purposes (e.g. gaps can be introduced in a first sequence for optimal alignment with a second sequence). The nucleotide or amino acid residues at each position are then compared. When a position in the first sequence is occupied by the same nucleotide or amino acid as the corresponding position in the second sequence, then the nucleotides or amino acids are identical at that position. The percent identity between the two sequences is a function of the number of identical positions shared by the sequences (i.e., % identity = number of identical positions /total number of positions in the reference sequence × 100). Typically, the sequence comparison is carried out over the length of the reference sequence, unless otherwise specified, e.g. to be only over the length of a coding sequence. For example, to assess whether a sequence is at least 95% identical to a reference sequence, the skilled person would carry out an alignment over the length of the reference sequence, and identify how many positions in the test sequence were identical to those of the reference sequence. If at least 95% of the positions are identical, the test sequence is at least 95% identical to the reference sequence. If the sequence is shorter than the reference sequence, the gaps or missing positions should be considered to be non-identical positions. If the sequence is longer than the reference sequence, the additional positions that are within the section aligned with the reference sequence should be considered to be non-identical positions.

The skilled person is aware of different computer programs that are available to determine the homology or identity between two sequences. For instance, a comparison of sequences and determination of percent identity between two sequences can be accomplished using a mathematical algorithm. In an embodiment, the percent identity between two amino acid or nucleic acid sequences is determined using the Needleman and Wunsch (1970) algorithm which has been incorporated into the GAP program in the Accelrys GCG software package (available at http://www.accelrys.com/products/gcg/), using either a Blosum 62 matrix or a PAM250 matrix, and a gap weight of 16, 14, 12, 10, 8, 6, or 4 and a length weight of 1, 2, 3, 4, 5, or 6.

The term *"about"* when referring to a value refers to a value that is within 20% (e.g. + 20% or - 20%), such as within 10%, within 5% or within 1% of the value.

The singular forms *"a", "an"* and *"the"* include plural references unless the context clearly dictates otherwise.

The term *"fermentation"* as used herein refers to the conversion of sugars to metabolic products by yeast. Typically, the term *"fermentation"* refers to *"alcoholic fermentation",* which is the conversion of sugars to alcohols, e.g. ethanol, by yeast. The fermentations described herein also lead to the production of other products including, but not limited to, yeast biomass and congeners (e.g. alcohols other than ethanol, ketones, aldehydes, esters and/or tannins).

All publications, patents and patent applications cited herein, whether *Supra* or *Infra,* are hereby incorporated by reference in their entirety.

### Examples

### The premise

The observation that exogenous addition of glucoamylase (GA) during high temperature grain Scotch whisky fermentation significantly increased ethanol yield by means of reducing residual maltotriose, was key for this invention. The suggested hypothesis was that GA could hydrolyze complex sugars, such as maltotriose, which are slow or difficult for yeast to assimilate, especially under high-temperature conditions (Tables 2 and 3). Therefore, we evaluated whether the production of secreted GA by Scotch strains could provide a significant advantage under stress conditions. We produced proof-of-concept engineered strains derived from GW and NT and expressing *STA1* from WY3711 (Krogerus *et al* (2019)), and the results confirmed that GA expression could benefit whisky strains (Tables 4 and 5). Since exogenous enzyme addition is not allowed in Scotch whisky production, we envisioned crossing a Scotch strain with a diastatic strain expressing STA1 glucoamylase to generate candidates with superior maltotriose and oligosaccharide utilization compared to both parent strains. The goal was to increase yield under high-temperature stress and improve sugar assimilation and yield under permissive temperature conditions.

In addition, as the Sta1 allows for the hydrolysis of maltotriose and oligosaccharides to simple sugars (glucose and maltose), which are assimilated faster as they are a preferred source of carbon, an improvement in fermentation kinetics was also targeted.

**Table 2. Ethanol and residual sugars at 72 h drop time of grain strains tested in malt wort fermentation under permissive temperature gradient 19-34°C and high temperature gradient 25-40°C. Results are means of triplicates ± SD.**

| Temperature ramp | **NT** | | **GW** | | **MW** | | **XP** | |
|---|---|---|---|---|---|---|---|---|
| | **Mean** | **Std Dev** | **Mean** | **Std Dev** | **Mean** | **Std Dev** | **Mean** | **Std Dev** |
| | **Ethanol (%v/v)** | | | | | | | |
| Permissive 19-34 °C | 7.73 | 0.04 | 7.9 | 0.06 | 8.16 | 0.03 | 8.31 | 0.06 |
| HIGH 25-40 °C | 6.36 | 0.13 | 7.07 | 0.07 | 6.81 | 0.01 | 7.86 | 0.05 |

| | **DP4(%w/v)** | | | | | | | |
|---|---|---|---|---|---|---|---|---|
| Permissive 19-34 °C | 1.67 | 0.02 | 1.69 | 0.02 | 1.68 | 0.01 | 1.39 | 0.02 |
| HIGH 25-40 °C | 1.93 | 0 | 1.96 | 0.02 | 1.93 | 0.01 | 1.57 | 0.01 |

| | **Maltotriose (%w/v)** | | | | | | | |
|---|---|---|---|---|---|---|---|---|
| Permissive 19-34 °C | 0.91 | 0.07 | 0.69 | 0.01 | 0.49 | 0 | 0.38 | 0 |
| HIGH 25-40 °C | 1.74 | 0.03 | 1.91 | 0.02 | 1.6 | 0.02 | 0.54 | 0.02 |

| | **Maltose (%w/v**) | | | | | | | |
|---|---|---|---|---|---|---|---|---|
| Permissive 19-34 °C | 0.34 | 0.02 | 0.25 | 0 | 0.24 | 0 | 0.26 | 0.01 |
| HIGH 25-40 °C | 1.71 | 0.19 | 0.35 | 0.01 | 1.17 | 0.05 | 0.4 | 0.03 |

| | **Glucose (%w/v)** | | | | | | | |
|---|---|---|---|---|---|---|---|---|
| Permissive 19-34 °C | 0.03 | 0.01 | 0.03 | 0 | 0.03 | 0 | 0.04 | 0.01 |
| HIGH 25-40 °C | 0.06 | 0 | 0.05 | 0 | 0.09 | 0.01 | 0.31 | 0.01 |

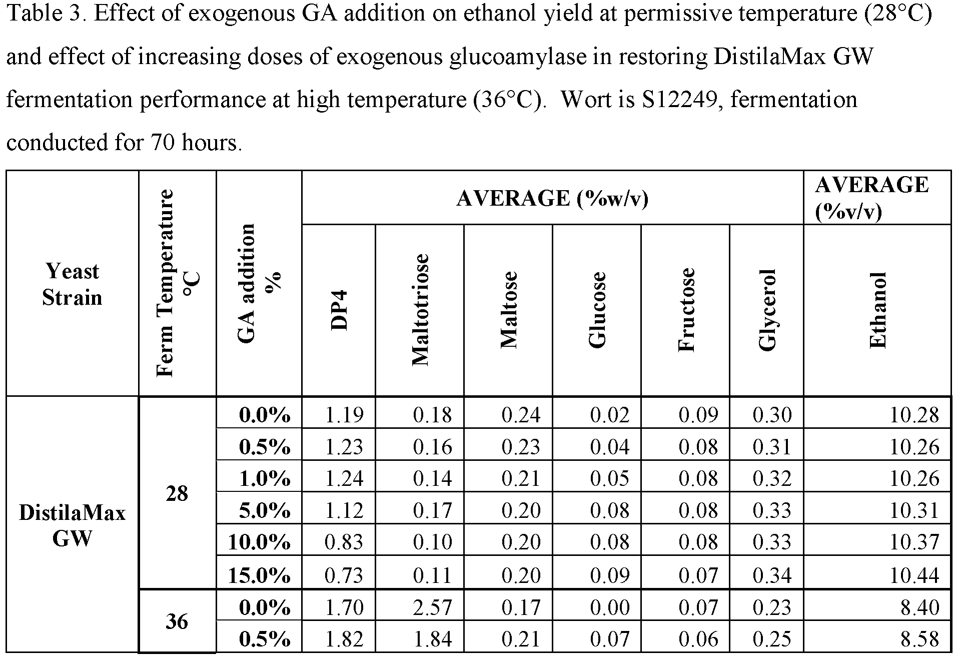

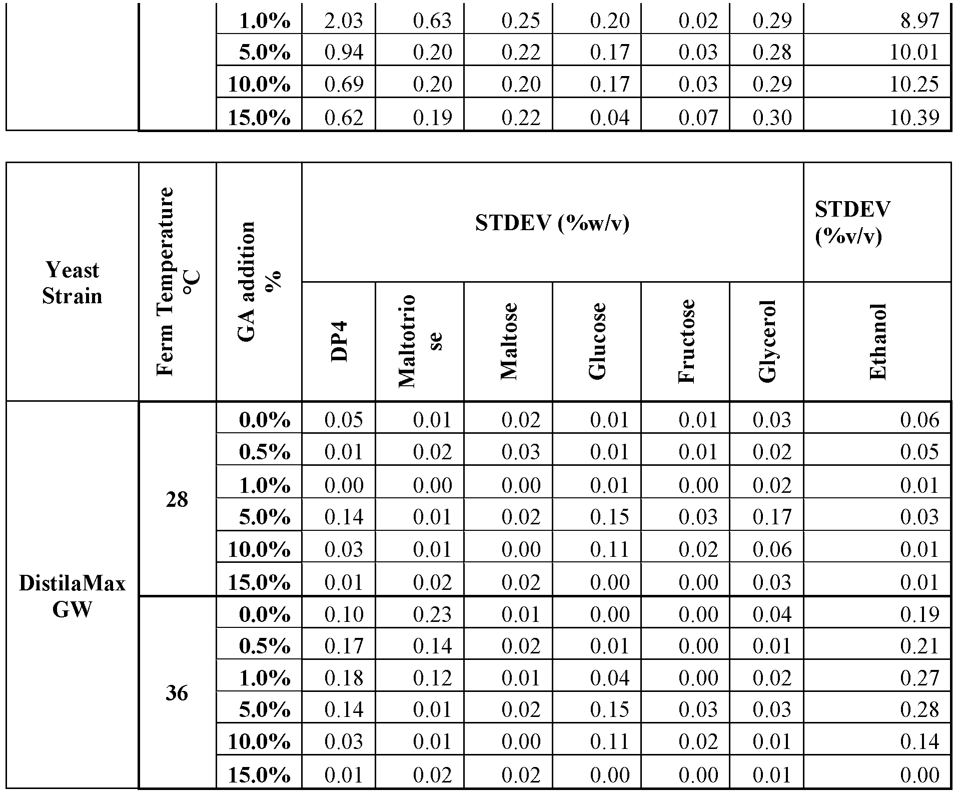

**Table 4. GA activity measured by glucose released from starch degradation. Supernatant from yeast culture was incubated with gelatinized starch for 20 h before glucose staining by DNS assay. GW, NT and their GMO variations as well as two diastatic strains, Belle Saison (BS) and DistilaMax^{®} XP were tested. 'Native' refers to a gene comprising the STA1 promoter, coding sequence and terminator sequence of strain WY3711 (Krogerus et al (2019)). 'Tef2' refers to a gene comprising the consistutive Tef2 promoter, and the STA1 coding sequence of strain WY3711 (Krogerus et al (2019)).**

| | **GW** | **NT** | **BS** | **XP** | **GW-native** | **GW-tef2** | **NT-native** | **NT-tef2** |
|---|---|---|---|---|---|---|---|---|
| OD at 600nm | 0.0161 | 0.0263 | 0.0595 | 0.0629 | 0.0739 | 0.2515 | 0.0328 | 0.3270 |
| STDEV | 0.0031 | 0.0012 | 0.0065 | 0.0047 | 0.0128 | 0.0030 | 0.0001 | 0.0084 |

**Table 5. PLC analysis of Scotch grain fermentation performed at high temperature (temperature increase from 20°C to 38°C) for 72 h. NT and GW controls were tested alongside diastatic strains (Belle Saison, XP) and NT and GW engineered strains expressing STA1 from strain WY3711 (Krogerus et al (2019)) with either the native STA1 promoter (STA1p) or the constitutive TEF2 promoter (TEF2p).**

| | MEAN (% w/v) | | | | |
|---|---|---|---|---|---|
| | Glucose | Maltose | Glycerol | Maltotriose | Ethanol |
| GW | 0.00 | 0.18 | 0.32 | 2.24 | 6.24 |
| NT | 0.00 | 0.73 | 0.28 | 1.31 | 6.37 |
| BS | 0.03 | 0.94 | 0.25 | 0.52 | 6.21 |
| XP | 0.06 | 3.63 | 0.31 | 0.53 | 5.15 |
| GW native | 0.02 | 0.26 | 0.32 | 1.73 | 6.43 |
| GW TEF2p | 0.03 | 0.20 | 0.34 | 0.18 | 7.01 |
| NT native | 0.03 | 0.87 | 0.29 | 1.04 | 6.41 |
| NT TEF2p | 0.22 | 0.77 | 0.31 | 0.31 | 6.63 |

| | STDEV (% w/v) | | | | |
|---|---|---|---|---|---|
| | Glucose | Maltose | Glycerol | Maltotriose | Ethanol |
| GW | 0.00 | 0.00 | 0.02 | 0.01 | 0.02 |
| NT | 0.00 | 0.01 | 0.02 | 0.01 | 0.02 |
| BS | 0.00 | 0.01 | 0.03 | 0.01 | 0.03 |
| XP | 0.00 | 0.00 | 0.02 | 0.01 | 0.02 |
| GW native | 0.00 | 0.00 | 0.01 | 0.01 | 0.01 |
| GW TEF2p | 0.00 | 0.00 | 0.01 | 0.00 | 0.01 |
| NT native | 0.00 | 0.00 | 0.02 | 0.01 | 0.02 |
| NT TEF2p | 0.02 | 0.00 | 0.04 | 0.00 | 0.04 |

### Hybridization strategy and selection

### Isolate A2 (GWxBS cross-mating)

First, we explored if we could hybridize a naturally GA expressing yeast with a whisky strain to achieve GA expression in a Scotch whisky strain. The hybrid A2 was selected from the crossing of DistilaMax GW and the STA1+ strain Belle Saison. Strain A2 performed better than both parental strains and the control DistilaMax NT under high temperature stress in the wort used for selection, displaying the positive characteristics of both GW (low residual maltose) and Belle Saison (low residual maltotriose). In application testing, the hybrid A2 showed better performance than NT in four out of five batches of wort under both permissive and high temperature conditions, showing superior ability to assimilate maltotriose compared to the controls (NT and GW). However, the hybrid A2 performance was negatively affected by high temperature in some substrates, highlighting the importance of substrate diversity during the selection process.

### Isolate B23-05 (NTxBS cross-mating)

In an attempt to combine the consistency of NT across different substrates (which was really understood and appreciated during these studies!) with the improved maltotriose utilization from Sta1, hybrids were generated by crossing the whisky strain DistilaMax NT (NT) and Belle Saison. In order to select for real hybrids between NT and Belle Saison we developed a pipeline with different steps to exclude false positives from our crossing experiment. After the mating experiment, populations were plated on YNB + Sm and Er to select for isolates with genetic backgrounds from both strains. Colonies growing on YNB-Sm/Er were then spread on YNB-Sm (confirmation of NT background) and inoculated in a maltodextrin based medium (growth selection for functional STA1 expression). According to the selection pipeline, isolates growing on these plates and in the maltodextrin medium were then selected for further analysis. Despite having several colonies growing on YNB-Sm/Er, only one isolate passed the second screening step of growth on YNB-Sm and growth in the maltodextrin medium.

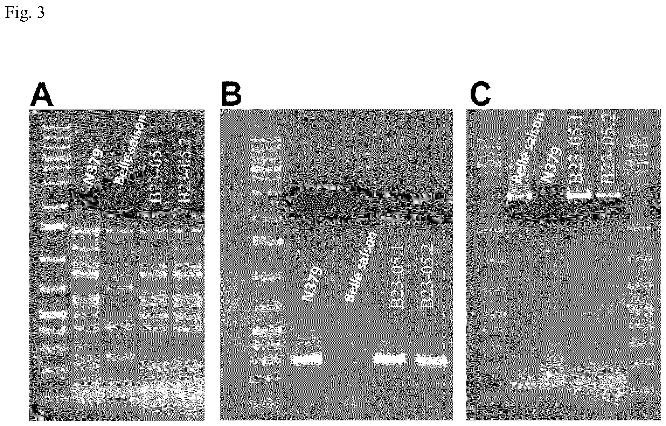

The isolate B23-05 was then spread on a YPD plate and two colonies (B23-05.1 and B23-05.2) were re-spread and used to make freezer stocks. We analysed both colonies as independent samples to confirm that the initial isolate was pure and to increase confidence in our further analysis results. First, we performed a delta PCR on both colonies and controls (Figure 3A). Both the colonies exhibited the same delta PCR profile, suggesting that the original isolate, B23-05, was pure. The delta profiles of B23-05.1 and B23-05.2 were similar to the profile of NT but with fewer bands (Figure 3). We then performed other analytic PCRs to confirm that B23-05.1 and B23-05.2 were real hybrids of NT and Belle Saison. The first PCR reaction was previously designed by the bioinformatic team to target DNA regions absent from Belle Saison gDNA (Figure 3B). As expected, NT showed signal but not Belle Saison. Both B23-05.1 and B23-05.2 isolates showed a signal, suggesting gDNA background from NT. The second PCR reaction targeted the STA1 gene (Figure 3C). As expected, Belle Saison displayed a signal for STA1 but not NT. Both B23-05.1 and B23-05.2 isolates displayed a signal for STA1 gene. These analyses confirmed that the hybrid B23-05 was a real hybrid from the crossing of NT with Belle Saison. Isolate B23-05 was then tested in Ankoms in eleven different Scotch grain worts (Table 6) under permissive and high temperature conditions. Strain B23-05 demonstrated better consistency and robustness in various wort substrates compared to NT.

### Isolate B24-01 (NTxBS cross-mating + self-mating + cross-mating)

To further improve the thermotolerance of hybrid B23-05 across the different grain substrates, new hybrids were generated by self-mating of hybrid B23-05 followed by mass mating of the top candidates from the previous step. The self-mating of B23-05 was performed to shuffle its genome. Top isolates were evaluated in grain worts under high temperature conditions. Top isolates were also tested in wort under permissive conditions and showed similar performance as B23-05. Using this method, we obtained three isolates with improved performance compared to B23-05 in grain worts under high temperature conditions, while maintaining similar performance in wort under permissive conditions. However, they were still not matching NT performance in these conditions. Next, a mass mated population was generated by mixing spores of B23-05 and three of the strains produced following the self-mating. The top isolates were then tested for fermentation performance in S13598 wort under high temperature stress. Three isolates were evaluated in minivials for fermentation performance in worts under permissive conditions to verify if they maintained the benefits of B23-05. Finally, using this strategy, we identified one isolate, B24-01, that matched or surpassed NT performance in certain worts under high temperatures, while maintaining yield improvement in worts under permissive temperatures. The isolate B24-01, was characterized in Ankoms in twelve different Scotch grain worts (Table 6) under permissive and high temperature conditions. The strain B24-01 displayed better performance and kinetics than DistilaMax NT in all the grain wort substrates tested. In addition, B24-01 exhibited similar performance and faster kinetics than XP in malt wort, suggesting potential applicability for process improvement in both grain and malt mashes. Medium scale fermentations and distillation were performed to generate enough volume of distillates for chemical and sensory evaluation. Overall, the two strains produced different congener profiles in both substrates tested. In one grain substrate, hybrid B24-01 produced higher amounts of acetaldehyde, acetal, isoamyl acetate and ethyl esters (excluding ethyl acetate) compared to NT (Table 15). In the malt wort substrate, B24-01 produced higher concentration of acetate and ethyl esters (excluding ethyl butyrate and hexanoate) compared to NT and lower or comparable concentrations of fusel alcohols (Table 16). Triangle discrimination testing was used to determine if the distillates from the two strains tested under the same conditions were similar or different (n=15). The aromatic profiles of the NMS distillates from the two strains in the grain substrate were comparable (p > 0.05), while the aromatic profiles of the NMS distillates from the two strains in the malt substrate were found to be different (p < 0.05). The DistilaMax NT and B24-01 distillates from the grain substrate were both described as having fruity, grainy, woody and nutty attributes. Both distillates from the malt substrate were described as fruity, cereal and sweet, with the B24-01 distillate having a more intense aroma than NT.

### Methods

### Yeast strain crossing

After generating spontaneous mutants of NT and Belle Saison with resistance to selective agents, sporulation of NT and Belle Saison was induced in sporulation medium (1 g/L yeast extract, 10 g/L potassium acetate, 0.5 g/L glucose) for 6 days at 23°C. Spores were prepared with zymolyase, mixed together in a small volume of rich media, incubated at 30°C overnight, and then spread on YNB agar plates for selection. Colonies that grew were streaked again on a selective medium. Single colonies were screened in maltodextrin-based medium.

### Yeast self-mating

Sporulation of B23-05 was induced in sporulation medium (1 g/L yeast extract, 10 g/L potassium acetate, 0.5 g/L glucose) for 6 days at 23°C. Spores were prepared with zymolyase and spores were then incubated in a small volume of rich media overnight. The culture was then pitched in fresh rich medium and incubated at 30°C overnight. Freezer stocks were produced by adding glycerol (25% final) to the saturated YPD culture.

### Yeast mass mating

Sporulation of B23-05 and three strains produced following the self-mating was induced in sporulation medium (1 g/L yeast extract, 10 g/L potassium acetate, 0.5 g/L glucose) for 6 days at 23°C. Spores were prepared with zymolyase and spores were incubated in a small volume of rich medium at 30°C overnight. The culture was then pitched in 5 ml of rich medium and incubated at 30°C overnight. Freezer stocks were produced by adding glycerol (25% final) to the saturated YPD culture.

### Growth assay in maltodextrin medium

Isolates were precultured in rich medium with glucose, normalized to equivalent cell density, and inoculated into rich media with maltodextrin as the carbon source (2 g/L yeast extract, 4 g/L peptone, 20 g/L maltodextrin; sterilized by filtration). Growth was monitored over time. Colonies that grew better than controls were then analyzed by PCR.

### Sample worts

**Table 6: list of worts. Description of the samples, including original gravity (OG), free amino nitrogen (FAN) content, and pH.**

| **Sample ID** | **Grain Wort Type** | **Malt Inclusion** | **Backset Inclusion** | **Original Gravity (OG)** | **pH** | **FAN (ppm)** |
|---|---|---|---|---|---|---|
| S12795 | Wheat | Yes | Yes | 1.067 | 4.96 | 83 |
| S12249 | Maize | Yes | No | 1.074 | 4.67 | 167 |
| S12797 | Wheat | Yes | No | 1.062 | 5.61 | 58 |
| S13612 | Wheat | Yes | No | 1.071 | 4.77 | 69 |
| S13613 | Wheat | Yes | Yes | 1.066 | 5.23 | 89 |
| S13597 | Wheat | Yes | Yes | 1.062 | 5.1 | 98 |
| S13598 | Maize | Yes | No | 1.065 | 5.32 | 96 |
| S14006 | Wheat | Yes | No | 1.062 | 4.79 | 85 |
| S14181 | Wheat | Yes | Yes | 1.077 | 4.96 | 116 |
| S14398 | Maize | Yes | No | 1.074 | 4.85 | 227 |
| S14399 | Wheat | Yes | Yes | 1.060 | 5.05 | 60 |
| S15799 | Wheat | Yes | No | 1.074 | 6.61 | 93 |
| S15777 | 100% malted barley | | No | 1.079 | 5.27 | 256 |

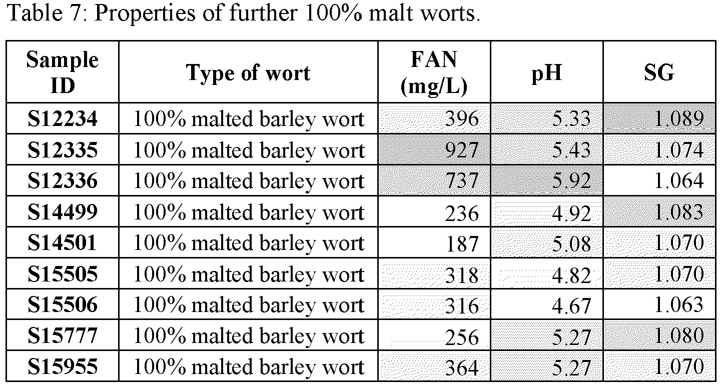

### PCR analysis

PCR fingerprinting of the putative hybrid and its parental strains, as well as PCR with primers targeting specific sequences present or absent in parental strains, was performed on genomic DNA.

### Screening in synthetic medium

Isolates were precultured in rich medium with glucose and then inoculated into synthetic medium for whisky (124 g/L maltose, 60 g/L maltodextrin, 1 mL/L ethanol anhydrous, 0.625 mLl/L glycerol, 3 mL/L lactic acid, 2 g/L yeast extract, 4 g/L peptone, pH adjusted to 5.9). Synthetic medium (3 mL) was distributed in minivials using a repeater pipette. 100 µl of saturated YPD culture was then used to inoculate the synthetic medium with yeast. Minivials were incubated without agitation at 30°C for 72 h. Minivials weight was measured at different time point to monitor CO2 released by the fermentation.

### SIL Scotch-type fermentations: selection of candidates for complete characterization

The two colonies from the hybrid B23-05 and controls were tested in Scotch-type whisky fermentations using various worts. The temperature was adjusted for permissive or high temperature conditions. For all fermentations, 3 g of whisky wort was aliquoted in minivials and 100 µl of saturated yeast preculture was used to inoculate samples. Fermentations were performed without agitation for 72 h. Once the fermentation was finished, the fermented wort was analyzed by HPLC.

### Ankom Scotch-type fermentations: new strain characterization

Scaled-down Scotch grain and malt fermentations were performed to evaluate fermentation performance and kinetics of the yeast candidates in the different wort substrates. Fermentations were carried out in triplicate in 30 g (100 mL Ankoms, see following section) of wort substrates (Tables 6 and 7). Yeast was pitched at 1.0 gDCW/L (grams of dry cell weight per liter) from overnight YPD cultures. Fermentations were carried out for 70 h (grain) and 92 h (malt) with no agitation and incubated using the temperatures ramps 20-30°C and 18-36°C for grain, and 18-28°C for malt (Table 14). The 20-30°C ramp is referred to as permissive temperature and the final temperature of 30°C is reached after 29 h. The 18-36°C ramp is referred to as high temperature and the temperature goes up to 36°C after 35.5 h of fermentation. The 18-28°C ramp is referred to malt permissive temperature profile and the final temperature of 28°C is reached after 20 h. The hybrid strains B24-01 was compared to the current commercial strain DistilaMax NT in twelve grain substrates and to DistilaMax NT and DistilaMax XP in malt wort. Analysis at the end of fermentation included: fermentation kinetics, HPLC, pH, density, and final gravity.

### ANKOM Kinetic Analysis

Ankom RF modules (Ankom Technology, NY, USA) were used to monitor gas evolution during fermentation. Each module consists of an RF sensor bottle cap equipped with a valve that releases the gas produced during fermentation, which was set up to take pressure measurements every 2 min. As the metabolized sugars are converted to ethanol and CO2, gas production is used to monitor the progression of the fermentation. Using JMP 17.0 (SAS Institute Inc.), the fermentation rate over time was extrapolated from the cumulative pressure (CP) plot by fitting average data points with a neural non-linear regression (Neural Network Function in JMP), from which the derivative (dCP/dt) was plotted against time. Vmax (mbar/h) was defined as the maximum fermentation rate (dCP/dt) achieved throughout the fermentation. The fermentation lag phase was determined by extrapolating the tangent to the fermentation curve at Vmax back to y=0. The time of completion (TC) was the time required for the fermentation rate to decrease below 20 mbar/h.

### HPLC Analysis

Sampling for HPLC analysis of ethanol, residual sugars, organic acids, and glycerol was performed at 70 h and 92 h drop time. Fermentation samples (ca. 2 mL) were spun down at 13,000 rpm by microcentrifuge (Eppendorf 5425, ON, Canada), then filtered through a 0.22 µm syringe-driven filter (Millipore, Cork, Ireland) and analyzed by HPLC (Waters Chromatography Division, Milford, MA, USA) using a 2414 refractive index detector at 45°C. For ethanol, glucose, DP3, DP4, maltose, glycerol and organic acids quantification, separation was achieved using a 150 x 7.8 mm Phenomenex-Rezex ROA-Organic acid column preceded by a KJO-4282 guard column (Phenomenex, Torrance, CA, USA). A mobile phase of sulfuric acid (0.01 N) was used with a flow rate of 0.6 mL/min and a column temperature of 65°C.

### Statistical Analysis of fermentation data

All statistical analysis were performed using the JMP 17.0 software. Error bars indicate standard deviation (SD). Statistical significance of ethanol yield comparison from drop fermentations was assessed by one-way ANOVA, Student's t test, Dunnet's test and Tukey test. Significance was determined at the 95 % confidence interval unless otherwise stated.

### Medium-scale Scotch whisky-type fermentations to generate distillates for chemical and sensory analysis

Medium-scale Scotch grain and malt fermentations were carried out in 600 g of wort substrates: Malt wort S15777 (2x 600 g) and grain wort S14181 (1x 600 g). Yeast was pitched at 1.0 gDCW/L (grams of dry cell weight per liter) from overnight YPD cultures. Grain fermentations were carried out for 70 h with no agitation and using the temperatures ramp 20-30°C. Malt fermentations were carried out for 96 h with no agitation and using the temperatures ramp 18-28°C. The strains were compared to the current commercial strain DistilaMax NT. Analysis at the end of fermentation included HPLC, fermentation kinetics pH and final gravity.

### Medium-scale double distillation to generate distillates for chemical and sensory analysis

Medium-scale distillations for sensory analysis were performed using a double glass distillation system. To perform the stripping run, a 1 L round-bottom flask (RBF) was attached to a lyne arm followed by a condenser. A volume of 500 mL of wash was loaded into the 1 L RBF and a total of 17 g of copper wool was used at various points throughout the system. The condenser was chilled using a temperature-controlled recirculation system, set to 10°C (VWR^{®} Recirculating chiller). The heating source for the RBF was a 1 L heating and stirring mantle (JOANLAB^{®}). The temperature was set at 2 and stirring level at low until the end of the distillation. Approximately 190 mL of low wines (LW) was collected.

For the spirit run, a 500 mL RBF was attached to a 30 cm straight column, followed by a lyne arm and condenser. A volume of 160 mL of LW was loaded and 20 g of copper wool were used. The heating source was a 1 L heating and stirring mantle (JOANLAB^{®}). The temperature was set at 1 until the end of the distillation. The heads cut was performed after 1 mL, then 24 mL of NMS (new make spirit) were collected. Tails were not collected.

### Gas chromatography

Distillate ethanol strength was measured using a DMA35 density meter (Anton-Paar Canada Inc, QC; LBDS SOP 079). Congener quantification was performed using an Agilent 7820A gas chromatography (GC) system coupled with a 7697A headspace (HS) auto sampler and equipped with a flame ionization detector (FID). The headspace heating zone was maintained at 80°C, the loop at 110°C and the transfer line at 120°C. A CP-Wax 57 CB Agilent column (50 m x 0.25 mm x 0.2 µm) was used for chromatography separation. The carrier gas was high purity hydrogen with a constant flow rate of 4 mL/min. The injector temperature was set at 220°C and a split ratio of 30:1 was used. The oven temperature was set at an initial temperature of 40°C, held for 2 min, raised at 10°C/min to 120°C, held for 10 min, raised at 70°C/min to 200°C and held for 5 min for a total of 26 min. The detector temperature was 300°C. Samples were analyzed using the 40 %ABV HS-tailored calibration curve. The LW were loaded at strength (25-30 %ABV), while NMS (all > 85 %ABV) were diluted to 40 %ABV. Internal standard (2-pentanol, 160 ppm) was added to samples prior to analysis. Samples were added (100 µL) to crimp-top headspace vials (20 mL, 23x75mm) containing 0.5 ± 0.05 g of sodium chloride. Data was normalized to g/100L of absolute alcohols (g/100L A.A.) for analysis.

### Sensory analysis

A triangle discrimination tests was performed for grain and malt distillates by fifteen panelists (n=15) from the LBDS team. Panelists were presented with two sets of three anonymized glasses filled with the NMS from either NT or B24-01 and adjusted to 20 %ABV with mineral water. The panelists were asked to nose the samples and select which sample was different. A binomial distribution test was conducted using JMP 17.0 software to determine the statistical significance of the results. Significance was determined within a 95% confidence interval.

### Results

The best performing strain, B24-01, was characterized in twelve grain wort substrates (Table 6), under both permissive and high temperature conditions. The isolate B24-01 showed comparable or better fermentation performance and faster kinetics than the commercial benchmark DistilaMax NT in all tests (Table 8). In addition, B24-01 was characterized in two malt wort substrates, where the strain exhibited similar performance and faster kinetics than DistilaMax XP, the most performant Lallemand strain in this substrate, suggesting potential applicability for process improvement in malt mash fermentation.

**Table 8. Comparison of the fermentation performance and kinetics of NT and B24-01 in twelve different grain wort substrates. Ethanol titer is expressed as a percentage relative to NT. Fermentation completion time is expressed as a percentage relative to NT. Results are presented as means ± SD of at least three replicates**

| Permissive temperature (20-30 °C) | | | | | | | | |
|---|---|---|---|---|---|---|---|---|
| **Substrate** | **% Ethanol vs NT** | | | | **% TC vs NT** | | | |
| | **Mean** | **Std Dev** | **Mean** | **Std Dev** | **Mean** | **Std Dev** | **Mean** | **Std Dev** |
| | **NT** | | **B24-01** | | **NT** | | **B24-01** | |
| **S12795** | 0.0% | 1.5% | 5.9% | 0.2% | 0.0% | 1.6% | -12.8% | 0.9% |
| **S12249** | 0.0% | 1.7% | 0.7% | 0.6% | NA | NA | NA | NA |
| **S12797** | 0.0% | 4.4% | 7.6% | 0.5% | 0.0% | 3.5% | -17.2% | 1.1% |
| **S13612** | 0.0% | 1.7% | 2.5% | 1.7% | -0.1% | 3.2% | -15.0% | 8.9% |
| **S13613** | 0.0% | 4.9% | 8.0% | 0.9% | 0.0% | 5.6% | -9.3% | 0.6% |
| **S13597** | 0.0% | 0.5% | 0.9% | 0.7% | 0.0% | 2.3% | -16.0% | 0.9% |
| **S13598** | 0.0% | 0.7% | 1.6% | 0.2% | 0.0% | 2.5% | -10.7% | 3.2% |
| **S14006** | 0.0% | 1.7% | -0.5% | 1.8% | 0.0% | 4.5% | -10.5% | 4.8% |
| **S14181** | 0.0% | 2.3% | 2.2% | 1.0% | 0.0% | 2.0% | -12.8% | 1.5% |
| **S14398** | 0.0% | 2.0% | 0.4% | 1.3% | 0.0% | 2.8% | -11.3% | 3.3% |
| **S14399** | 0.0% | 12.0% | 13.6% | 0.8% | 0.0% | 3.0% | -5.7% | 2.4% |
| **S15799** | 0.0% | 0.5% | 1.9% | 1.8% | 0.0% | 0.8% | -8.0% | 1.9% |

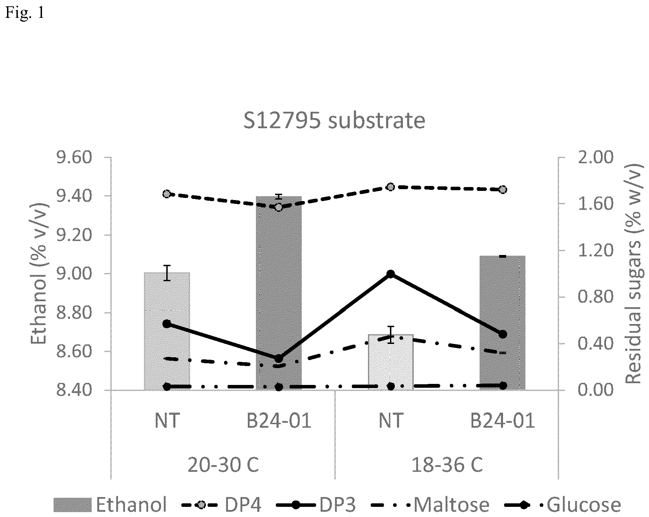

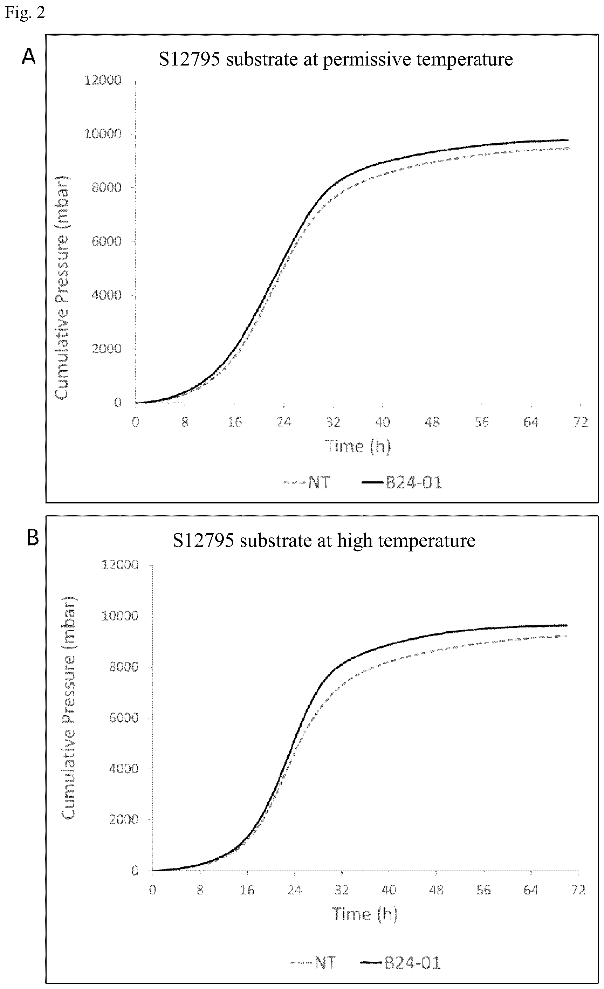

The novel strain B24-01 exhibits the following characteristics:
1. **Improved Thermotolerance:** Compared to NT, B24-01 produced higher ethanol under high-temperature stress in twelve different grain wort substrates (Tables 6, 8 and 10). This correlated with improved maltotriose (DP3) utilization (Tables 8 to 10) and, depending on the substrate, improved oligosaccharides (DP4) and maltose (DP2) utilization (Figure 1, Tables 9 and 10). Residual glucose was < 1 g/L in all fermentations (Tables 9 and 10).
2. **Comparable or Improved Fermentation Performance:** Compared to NT, B24-01 exhibited comparable or improved performance under permissive temperature in twelve different industrial grain wort substrates (Figure 1, Tables 6 and 8 to 10). The yeast B24-01 achieved higher ethanol yield due to improved DP3 and DP4 utilization (Figure 1, Tables 9 and 10). Residual maltose concentration was variable in B24-01 samples compared to NT (Figure 1, Tables 9 and 10), but generally higher than in NT samples (Tables 9 and 10). Residual glucose was < 1 g/L in all fermentations (Tables 9 and 10).
3. **Improved Fermentation Kinetics:** Under both permissive and high-temperature stress conditions, B24-01 showed improved fermentation kinetics compared to NT (Figure 2, Table 11). The strain B24-01 generally exhibited a longer lag phase than NT, but a faster maximum fermentation rate and a shorter time to fermentation completion (TC, Table 11).
4. **Performance and kinetics in malt substrate:** B24-01 exhibited similar performance and faster kinetics than DistilaMax XP, the Lallemand's most performant malt strain, in two malt worts, suggesting potential applicability for process improvement in malt mashes (Tables 12 and 13).
5. **Flavor Profile:** B24-01 maintains a typical Scotch whisky flavor profile with no major off-notes. The hybridization process did not negatively affect the chemical and sensory profile produced by the strain compared to the benchmark, which was a desirable characteristic (Tables 15 and 16).

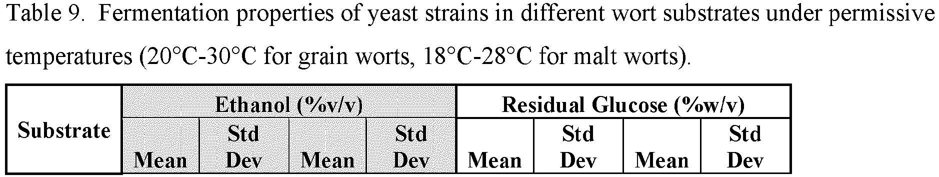

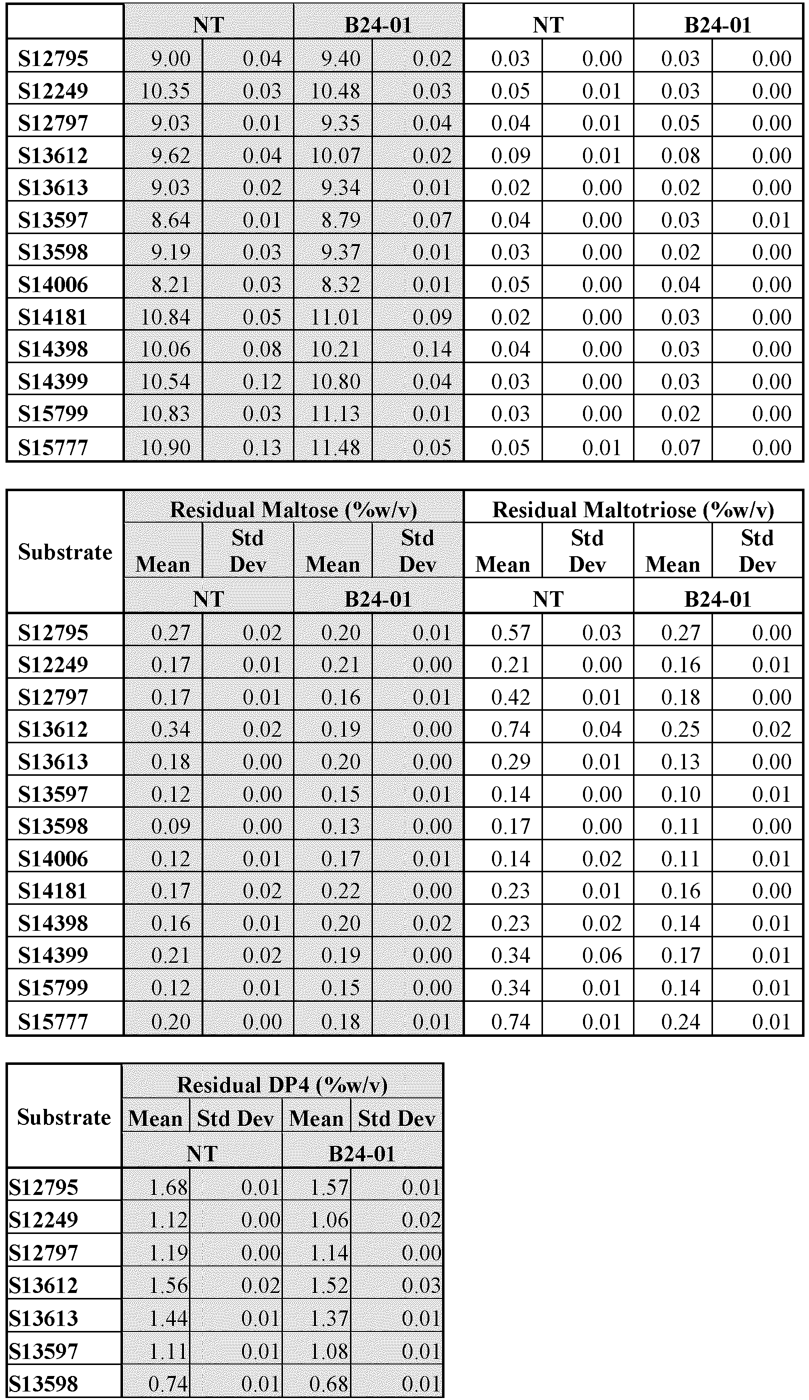

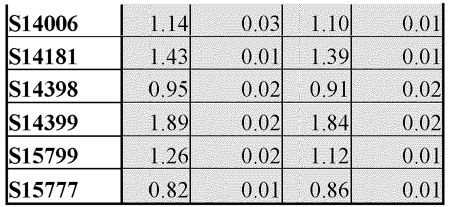

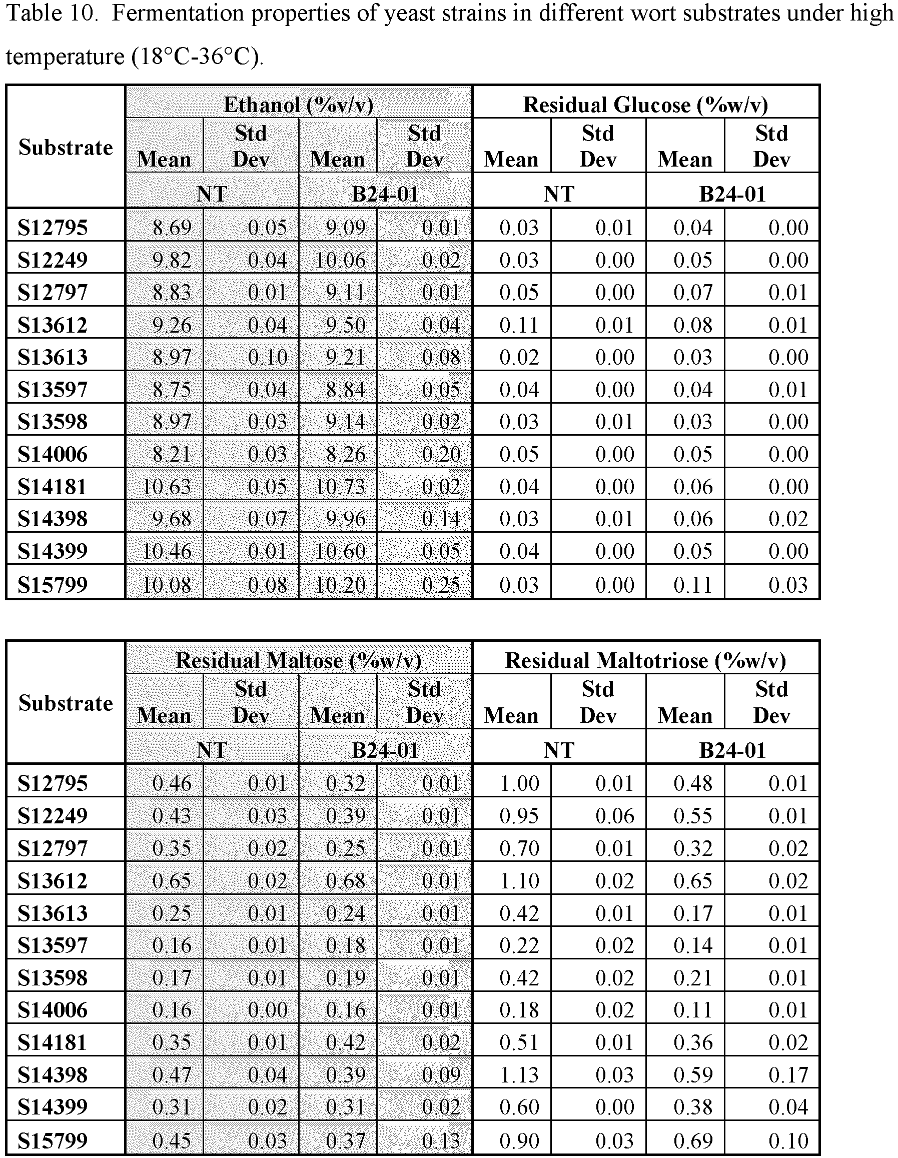

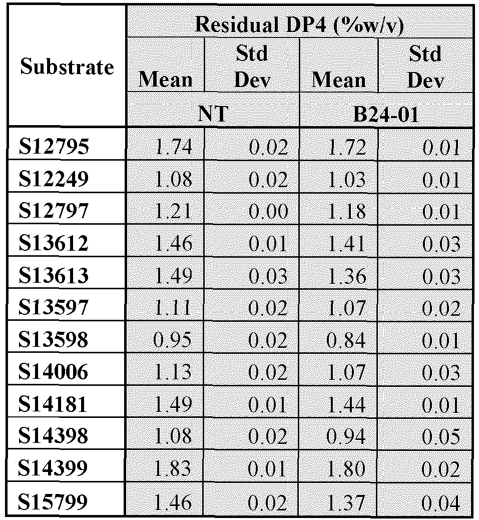

**Table 11. Time of fermentation completion (TC) expressed in hours from Scotch fermentations with NT and B24-01 yeast strains in different grain wort substrates.**

| **Substrate** | **TC (h) at permissive temperature (18-28 °C ramp)** | | | | **TC (h) at permissive temperature (20-30 °C ramp)** | | | |
|---|---|---|---|---|---|---|---|---|
| | **Mean** | **Std Dev** | **Mean** | **Std Dev** | **Mean** | **Std Dev** | **Mean** | **Std Dev** |
| | **NT** | | **B24-01** | | **NT** | | **B24-01** | |
| **S12795** | NA | NA | NA | NA | 68.81 | 0.7 | 58.89 | 0.84 |
| **S12249** | NA | NA | NA | NA | NA | NA | NA | NA |
| **S12797** | NA | NA | NA | NA | 63.12 | 0.62 | 51.94 | 0.92 |
| **S13612** | NA | NA | NA | NA | 67.54 | 1.69 | 60.78 | 1.22 |
| **S13613** | NA | NA | NA | NA | 60.5 | 0.05 | 52.33 | 0.26 |
| **S13597** | NA | NA | NA | NA | 55.01 | 0.68 | 44.97 | 0.55 |
| **S13598** | NA | NA | NA | NA | 53.62 | 1.05 | 47 | 1.33 |
| **S14006** | NA | NA | NA | NA | 54.69 | 1.4 | 47.04 | 2.01 |
| **S14181** | NA | NA | NA | NA | 53.2 | 0.49 | 45.73 | 0.68 |
| **S14398** | NA | NA | NA | NA | 59.04 | 0.66 | 54.19 | 1.98 |
| **S14399** | NA | NA | NA | NA | 56.84 | 0.53 | 53.02 | 1.29 |
| **S15799** | NA | NA | NA | NA | 64.33 | 0.58 | 58.33 | 0.58 |
| **S15777** | 86.67 | 0.58 | 76.67 | 1.53 | NA | NA | NA | NA |

| **Substrate** | **TC (h) at high temperature (18-36 °C ramp)** | | | |
|---|---|---|---|---|
| | **Mean** | **Std Dev** | **Mean** | **Std Dev** |
| | **NT** | | **B24-01** | |
| **S12795** | 63.61 | 1.17 | 55.27 | 0.38 |
| **S12249** | NA | NA | NA | NA |
| **S12797** | 63.01 | 0.88 | 49.8 | 0.26 |
| **S13612** | 64.17 | 0.94 | 50.09 | 0.68 |
| **S13613** | 60.19 | 0.6 | 50.71 | 0.41 |
| **S13597** | 51.91 | 0.36 | 43.37 | 0.47 |
| **S13598** | 53.6 | 1.07 | 49.16 | 0.07 |
| **S14006** | 51.19 | 1.68 | 44.39 | 3.22 |
| **S14181** | 53.89 | 0.38 | 46.24 | 0.8 |
| **S14398** | 56.39 | 2.65 | 50 | 1.35 |
| **S14399** | 56.7 | 0.85 | 51.32 | 0.91 |
| **S15799** | 65.33 | 0.58 | 61 | 1 |
| **S15777** | NA | NA | NA | NA |

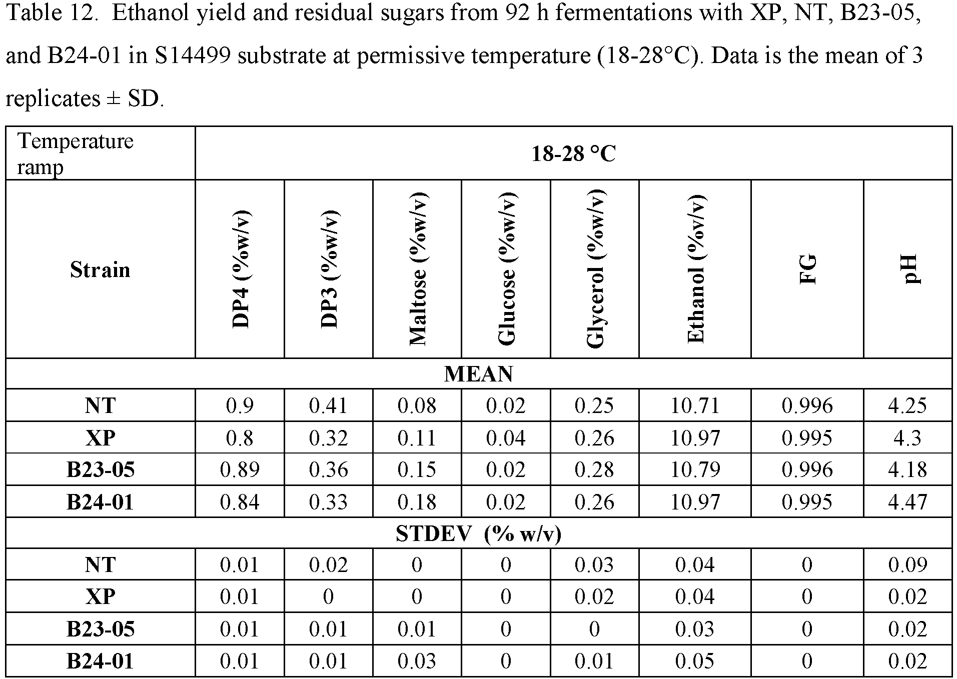

**Table 13. Time of fermentation completion (TC) and percent change in fermentation time from strains B23-05, B24-01 and DistilaMax XP compared to DistilaMax NT in S14499 wort at permissive temperature (18-28°C). Data is the mean of 3 replicates ± SD.**

| Temperature (°C) | Strain | TC (h) | | TC difference (%) | |
|---|---|---|---|---|---|
| | | MEAN | STDEV | MEAN | STDEV |
| 18-28 | NT | 67.5 | 3.2 | 0.0% | 0.0 |
| | B23-05 | 68.8 | 1.3 | 1.8% | 0.0 |
| | XP | 63.5 | 3.6 | -6.0% | 0.1 |
| | B24-01 | 60.8 | 2.0 | -9.9% | 0.0 |

**Table 14. Detailed profile of the 20-30°C (grain - permissive temperature), 18-36°C (grain - high temperature) and 18-28°C (malt - permissive temperature) temperature ramps.**

| **20-30 (°C Ramp Temp** | | | **18-36 °C Ramp Temp** | | | **18-28 °C Ramp Temp** | | |
|---|---|---|---|---|---|---|---|---|
| **Initial Temp** | **Final Temp** | **Time (h)** | **Initial Temp** | **Final Temp** | **Time (h)** | **Initial (°C)** | **Final (°C)** | **Time (h)** |
| 20 | 21 | 2 | 18 | 18.5 | 4 | 18 | 23 | 6 |
| 21 | 22 | 3 | 18.5 | 19.5 | 4 | 23 | 25 | 4 |
| 22 | 23 | 3 | 19.5 | 21 | 4 | 25 | 28 | 10 |
| 23 | 24 | 3 | 21 | 24.5 | 4 | 28 | | 50 |
| 24 | 25 | 3 | 24.5 | 29 | 4 | | | |
| 25 | 26 | 3 | 29 | 33 | 4 | | | |
| 26 | 27 | 3 | 33 | 34.5 | 4 | | | |
| 27 | 28 | 3 | 34.5 | 35.5 | 3 | | | |
| 28 | 29 | 3 | 35.5 | 36 | 3 | | | |
| 29 | 30 | 3 6 | 36 | | 36 | | | |
| 30 | | 41 | | | | | | |

**Table 15. Congener profile of spirit distillation (n=1) from medium-scale grain Scotch whisky fermentation with DistilaMax NT and hybrid B24-01 in wort S14181. Data is expressed in g/100L A.A.**

| **Grain Substrate: S14181** | | |
|---|---|---|
| **Congeners** | **Concentration(g/100L A.A)** | |
| | **NT (NMS)** | **B24-01 (NMS)** |
| **Acetaldehyde** | 22.7 ± 0.0 | **49.8** ± 0.0 |
| **Ethyl acetate** | 43.6 ± 0.0 | **40.4** ± 0.0 |
| **Acetal** | 1.8 ± 0.0 | 3.9 ± 0.0 |
| **Methanol** | 7.2 ± 0.0 | 7.8 ± 0.0 |
| **Isobutyl acetate** | <LOQ | <LOQ |
| **Ethyl butyrate** | <LOQ | <LOQ |
| **1-propanol** | 63.1 ± 0.0 | 64.5 ± 0.0 |
| **Isobutanol** | 79.4 ± 0.0 | 81.4 ± 0.0 |
| **1-butanol** | 1.0 ± 0.0 | 0.7 ± 0.0 |
| **2-Methyl-1-butanol** | 42.7 ± 0.0 | 43.3 ± 0.0 |
| **3-Methyl-1-butanol** | 140.4 ± 0.0 | 137.1 ± 0.0 |
| **Ethyl lactate** | <LOQ | <LOQ |
| **Isoamyl acetate** | 1.7 ± 0.0 | 2.8 ± 0.0 |
| **Ethyl hexanoate** | 0.6 ± 0.0 | 0.9 ± 0.0 |
| **Ethyl octanoate** | 1.5 ± 0.0 | 2.3 ± 0.0 |
| **Ethyl decanoate** | <LOQ | 0.9 ± 0.0 |
| **Phenethyl acetate** | <LOQ | <LOQ |

**Table 16. Congener profile of blended spirit distillations (n=2) from medium-scale malt whiskey fermentations with DistilaMax NT and hybrid B24-01 in wort S15777. Data is the mean of two replicates ± SD. Data is expressed in g/100L A.A.**

| **100 % Malt Substrate: S15777** | | |
|---|---|---|
| **Congeners** | **Concentration (g/100L A.A)** | |
| | **NT (NMS)** | **B24-01 (NMS)** |
| **Acetaldehyde** | 47.8 ± 5.3 | 52.0 ± 1.0 |
| **Ethyl acetate** | 106.4 ± 16.6 | 153.3 ± 2.4 |
| **Acetal** | 3.7 ± 0.6 | 4.4 ± 0.4 |
| **Methanol** | 4.6 ± 0.3 | 4.1 ± 0.1 |
| **Isobutyl acetate** | 0.6 ± 0.0 | 1.0 ± 0.0 |
| **Ethyl butyrate** | 0.7 ± 0.1 | 0.5 ± 0.0 |
| **1-propanol** | 44.7 ± 0.8 | 40.8 ± 1.6 |
| **Isobutanol** | 69.3 ± 0.9 | 62.7 ± 3.5 |
| **1-butanol** | 2.2 ± 0.1 | 1.6 ± 0.2 |
| **2-Methyl-1-butanol** | 43.3 ± 2.5 | 45.0 ± 6.7 |
| **3-Methyl-1-butanol** | 113.3 ± 10.4 | 147.4 ± 25.8 |
| **Ethyl lactate** | ND | ND |
| **Isoamyl acetate** | 15.8 ± 0.3 | 30.4 ± 0.4 |
| **Ethyl hexanoate** | 1.6 ± 0.0 | 1.4 ± 0.0 |
| **Ethyl octanoate** | 4.3 ± 0.1 | 6.9 ± 0.3 |
| **Ethyl decanoate** | 3.1 ± 1.2 | 5.6 ± 1.1 |
| **Phenethyl acetate** | ND | <LOQ |

### References

Alves 2008. Maltotriose active transport and fermentation by Saccharomyces cerevisiae on AGT1 permease.
Day 2002. Molecular analysis of maltotriose transport and utilization by Saccharomyces cerevisiae*.*
Dietvorst 2005. MTTI encodes a maltotriose transporter.
Krogerus 2019. A deletion in the STA1 promoter determines maltotriose and starch utilization in STA1 Saccharomyces cerevisiae strains.
Legras, J. L., & Karst, F. (2003). Optimisation of interdelta analysis for Saccharomyces cerevisiae strain characterisation. FEMS microbiology letters, 221(2), 249-255
Walker and Hill 2016. Saccharomyces cerevisiae in the production of whiskey.
Waymark and Hill 2021. The Influence of Yeast Strain on Whisky New Make.
Xufre et al (2011). Use of interdelta polymorphisms of Saccharomyces cerevisiae strains to monitor population evolution during wine fermentation. Journal of Industrial Microbiology and Biotechnology, 38(1), 127-132

### Further embodiments of the invention

1. A method of obtaining a hybrid yeast strain comprising crossing a first yeast strain with a second yeast strain that comprises a functional gene encoding an extracellular glucoamylase,
   to thereby produce the hybrid yeast strain,
   wherein the hybrid yeast strain, when compared to the first yeast strain, is capable of:
      (i) increasing trisaccharide utilisation;
      (ii) increasing maltose utilisation;
      (iii) producing more ethanol;
      (iv) reducing total fermentation time;
      (v) increasing maximum fermentation rate; and/or
      (vi) increasing oligosaccharide utilisation;
      during an alcoholic fermentation of a whisk(e)y wort.
2. The method of embodiment 1, wherein the first and/or second yeast strain is a *Saccharomyces* sp. strain.
3. The method of embodiment 1 or 2, wherein the first and/or second yeast strain is a *Saccharomyces cerevisiae* strain.
4. The method of any one of the preceding embodiments, wherein the first and second yeast strains are not genetically engineered organisms.
5. The method of any one of the preceding embodiments, wherein first yeast strain is a whisk(e)y yeast strain.
6. The method of any one of the preceding embodiments, wherein the first yeast strain (a) does not comprise a functional gene encoding an extracellular glucoamylase and/or (b) comprises a functional gene encoding a maltose permease and/or maltotriose permease, optionally wherein the functional gene encoding the maltose permease and/or the maltotriose permease is selected from AGT1 or MTY1.
7. The method of any one of the preceding embodiments, wherein the second yeast strain is a *S.cerevisiae* var *diastaticus* strain.
8. The method of any one of the preceding embodiments, wherein the functional glucoamylase gene is STA1, STA2 or STA3, preferably STA1.
9. The method of any one of the preceding embodiments, wherein the trisaccharide is maltotriose.
10. The method of any one of the preceding embodiments, wherein the hybrid yeast strain, when compared to the first yeast strain, is capable of maintaining the congener profile of the fermentation product.
11. The method of any one of the preceding embodiments, wherein the whisk(e)y wort is a grain whisk(e)y wort.
12. The method of any one of the preceding embodiments, wherein the whisk(e)y wort is a malt whisk(e)y wort.
13. The method of any one of the preceding embodiments, wherein the alcoholic fermentation is at least partially performed at a temperature of 35°C or greater.
14. The method of any one of the preceding embodiments, wherein the alcoholic fermentation is at least partially performed at a temperature of 35°C or greater for at least 6 hours.
15. The method of any one of the preceding embodiments, wherein the crossing step comprises cross-mating of the first yeast strain and second yeast strain to produce an intermediate hybrid yeast strain, and
   (a) self-mating the intermediate hybrid yeast strain; and/or
   (b) mass mating the intermediate hybrid yeast strain(s);
   to thereby produce the hybrid yeast strain.
16. The method of any one of the preceding embodiments, wherein the method comprises screening and selecting a yeast strain produced by the crossing having one or more of the characteristics selected from (i) to (vi) above.
17. A hybrid yeast strain obtained from, or obtainable by, the method of any one of the preceding embodiments.
18. A hybrid yeast strain deposited with the Collection Nationale de Cultures de Microorganismes (CNCM) Patent Depository, on 26 November 2024, with the Deposit No. CNCM I-6150.
19. A hybrid yeast strain which is a hybrid of a whisk(e)y yeast strain and a yeast strain that comprises a functional gene encoding an extracellular glucoamylase, wherein the hybrid yeast strain is capable of utilising maltotriose, optionally wherein the hybrid yeast strain is a Saccharomyces cerevisiae x Saccharomyces cerevisiae var. diastaticus hybrid.
20. The hybrid yeast strain of any one of embodiments 17 to 19, comprising:
   (a) a functional gene encoding an extracellular glucoamylase, optionally wherein the functional glucoamylase gene is STA1, STA2 or STA3, preferably STA1; and/or
   (b) a functional gene encoding a maltose permease and/or a maltotriose permease, optionally wherein the functional gene encoding the maltose permease and/or the maltotriose permease is AGT1 or MTY1.
21. The hybrid yeast strain of any one of embodiments 17 to 20, which is capable of:
   (a) achieving less than 1 % w/v residual glucose, such as less than 0.5 % w/v, 0.1 % w/v or 0.05 % w/v residual glucose;
   (b) achieving less than about 0.8 % w/v residual maltose, such as less than about 0.7% w/v, less than about 0.6% w/v, less than about 0.5% w/v, or less than about 0.4% w/v;
   (c) achieving less than about 0.8% w/v, such as less than about 0.7% w/v, less than about 0.6% w/v, less than about 0.5% w/v, less than about 0.4% w/v or less than about 0.3% w/v residual maltotriose; and/or
   (d) achieving at least about 8% v/v alcohol, such as at least about 8.5% v/v, or at least about 9% v/v,
   following the fermentation of whisk(e)y wort.
22. A method of producing a derivative yeast strain of the hybrid yeast strain of any one of embodiments 17 to 21, comprising crossing the hybrid yeast strain with a further yeast strain to produce a derivative yeast strain which, when compared to an ancestral first yeast strain, is capable of:
   (i) increasing trisaccharide utilisation;
   (ii) increasing maltose utilisation;
   (iii) producing more ethanol;
   (iv) reducing total fermentation time;
   (v) increasing maximum fermentation rate; and/or
   (vi) increasing oligosaccharide utilisation;
   during an alcoholic fermentation of a whisk(e)y wort.
23. The method of embodiment 22, wherein the method further comprises crossing the derivative yeast strain with a further yeast strain to produce a further derivative yeast strain which, when compared to an ancestral first yeast strain, is capable of:
   (i) increasing trisaccharide utilisation;
   (ii) increasing maltose utilisation;
   (iii) producing more ethanol;
   (iv) reducing total fermentation time;
   (v) increasing maximum fermentation rate; and/or
   (vi) increasing oligosaccharide utilisation;

   during an alcoholic fermentation of a whisk(e)y wort,
   optionally wherein the method is repeated one or more times.
24. The method of embodiment 22 or 23, wherein the crossing is performed by cross-mating, self-mating and/or mass-mating.
25. A derivative yeast strain obtained, or obtainable by, the method of any one of the preceding embodiments.
26. The derivative yeast strain of embodiment 25, comprising:
   (a) a functional gene encoding an extracellular glucoamylase, optionally wherein the functional glucoamylase gene is STA1, STA2 or STA3, preferably STA1; and/or
   (b) a functional gene encoding a maltose permease and/or a maltotriose permease, optionally wherein the functional gene encoding the maltose permease and/or the maltotriose permease is AGT1 or MTY1.
27. The derivative yeast strain of embodiment 25 or 26, which is capable of:
   (a) achieving less than 1 % w/v residual glucose, such as less than 0.5 % w/v, 0.1 % w/v or 0.05 % w/v residual glucose;
   (b) achieving less than about 0.8 % w/v residual maltose, such as less than about 0.7% w/v, less than about 0.6% w/v, less than about 0.5% w/v, or less than about 0.4% w/v;
   (c) achieving less than about 0.8% w/v, such as less than about 0.7% w/v, less than about 0.6% w/v, less than about 0.5% w/v, less than about 0.4% w/v or less than about 0.3% w/v residual maltotriose; and/or
   (d) achieving at least about 8% v/v alcohol, such as at least about 8.5% v/v, or at least about 9% v/v,
   following the fermentation of whisk(e)y wort.
28. A fermentation medium comprising a whisk(e)y wort and a hybrid or derivative yeast strain according to any one of embodiments 17 to 21 and 25 to 27.
29. A method for producing a fermented product, comprising:
   (a) optionally performing the method of any one of embodiments 1 to 16 and 22 to 24;
   (b) fermenting a wort or a must with the hybrid yeast strain of (a) or the hybrid or derivative yeast strain according to any one of embodiments 17 to 21 and 25 to 27.
30. The method of embodiment 29, which further comprises distilling and/or maturing the product produced in step (b).
31. The method of embodiment 29 or 30, wherein the wort or must is a whisk(e)y wort and the fermented product is a whisk(e)y, optionally wherein the wort is a grain whisk(e)y wort or a malt whisk(e)y wort.
32. The method of any one of embodiments 29 to 31, wherein the fermentation is at least partially performed at a temperature of 35°C or greater, optionally wherein the fermentation is at least partially performed at a temperature of 35°C or greater for at least 6 hours.

## Claims

1. A method of obtaining a hybrid yeast strain comprising crossing a first yeast strain with a second yeast strain that comprises a functional gene encoding an extracellular glucoamylase,
to thereby produce the hybrid yeast strain,
wherein the hybrid yeast strain, when compared to the first yeast strain, is capable of:
(i) increasing trisaccharide utilisation;
(ii) increasing maltose utilisation;
(iii) producing more ethanol;
(iv) reducing total fermentation time;
(v) increasing maximum fermentation rate; and/or
(vi) increasing oligosaccharide utilisation;
during an alcoholic fermentation of a whisk(e)y wort.

2. The method of claim 1, wherein the first and/or second yeast strain:
(a) is a *Saccharomyces* sp. strain;
(b) is a *Saccharomyces cerevisiae* strain; and/or
(c) are not genetically engineered organisms.

3. The method of claim 1 or 2, wherein first yeast strain:
(a) is a whisk(e)y yeast strain;
(b) does not comprise a functional gene encoding an extracellular glucoamylase; and/or
(c) comprises a functional gene encoding a maltose permease and/or maltotriose permease, optionally wherein the functional gene encoding the maltose permease and/or the maltotriose permease is selected from *AGT1* or *MTY1.*

4. The method of any one of the preceding claims, wherein:
(a) the second yeast strain is a *S.cerevisiae* var *diastaticus* strain; and/or
(b) the functional glucoamylase gene is *STA1, STA2* or *STA3,* preferably *STA1.*

5. The method of any one of the preceding claims, wherein:
(a) the trisaccharide is maltotriose; and/or
(b) the hybrid yeast strain, when compared to the first yeast strain, is capable of maintaining the congener profile of the fermentation product.

6. The method of any one of the preceding claims, wherein:
(a) the whisk(e)y wort is a grain whisk(e)y wort; and/or
(b) the alcoholic fermentation is at least partially performed at a temperature of 35°C or greater, optionally wherein the alcoholic fermentation is at least partially performed at a temperature of 35°C or greater for at least 6 hours.

7. The method of any one of the preceding claims, wherein:
(a) the crossing step comprises cross-mating of the first yeast strain and second yeast strain to produce an intermediate hybrid yeast strain, and
(I) self-mating the intermediate hybrid yeast strain; and/or
(II) mass mating the intermediate hybrid yeast strain(s);
to thereby produce the hybrid yeast strain; and/or
(b) the method comprises screening and selecting a yeast strain produced by the crossing having one or more of the characteristics selected from (i) to (vi) above.

8. A hybrid yeast strain obtained from, or obtainable by, the method of any one of the preceding claims.

9. A hybrid yeast strain:
(a) deposited with the Collection Nationale de Cultures de Microorganismes (CNCM) Patent Depository, on 26 November 2024, with the Deposit No. CNCM I-6150; or
(b) which is a hybrid of a whisk(e)y yeast strain and a yeast strain that comprises a functional gene encoding an extracellular glucoamylase, wherein the hybrid yeast strain is capable of utilising maltotriose, optionally wherein the hybrid yeast strain is a *Saccharomyces cerevisiae x Saccharomyces cerevisiae* var. *diastaticus* hybrid.

10. A method of producing a derivative yeast strain of the hybrid yeast strain of claim 8 or 9, comprising crossing the hybrid yeast strain with a further yeast strain to produce a derivative yeast strain which, when compared to an ancestral first yeast strain, is capable of:
(i) increasing trisaccharide utilisation;
(ii) increasing maltose utilisation;
(iii) producing more ethanol;
(iv) reducing total fermentation time;
(v) increasing maximum fermentation rate; and/or
(vi) increasing oligosaccharide utilisation;
during an alcoholic fermentation of a whisk(e)y wort.

11. The method of claim 10, wherein:
(a) the method further comprises crossing the derivative yeast strain with a further yeast strain to produce a further derivative yeast strain which, when compared to an ancestral first yeast strain, is capable of:
(i) increasing trisaccharide utilisation;
(ii) increasing maltose utilisation;
(iii) producing more ethanol;
(iv) reducing total fermentation time;
(v) increasing maximum fermentation rate; and/or
(vi) increasing oligosaccharide utilisation;
during an alcoholic fermentation of a whisk(e)y wort,
optionally wherein the method is repeated one or more times; and/or
(b) the crossing is performed by cross-mating, self-mating and/or mass-mating.

12. A derivative yeast strain obtained, or obtainable by, the method of claim 10 or 11.

13. The hybrid yeast strain of claim 8 or 9, or the derivative yeast strain of claim 13, which:
(a) comprises a functional gene encoding an extracellular glucoamylase, optionally wherein the functional glucoamylase gene is STA1, STA2 or STA3, preferably STA1;
(b) comprises a functional gene encoding a maltose permease and/or a maltotriose permease, optionally wherein the functional gene encoding the maltose permease and/or the maltotriose permease is AGT1 or MTY1; and/or
(c) is capable of:
(a) achieving less than 1 % w/v residual glucose, such as less than 0.5 % w/v, 0.1 % w/v or 0.05 % w/v residual glucose;
(b) achieving less than about 0.8 % w/v residual maltose, such as less than about 0.7% w/v, less than about 0.6% w/v, less than about 0.5% w/v, or less than about 0.4% w/v;
(c) achieving less than about 0.8% w/v, such as less than about 0.7% w/v, less than about 0.6% w/v, less than about 0.5% w/v, less than about 0.4% w/v or less than about 0.3% w/v residual maltotriose; and/or
(d) achieving at least about 8% v/v alcohol, such as at least about 8.5% v/v, or at least about 9% v/v,
following the fermentation of whisk(e)y wort.

14. A fermentation medium comprising a whisk(e)y wort and a hybrid or derivative yeast strain according to any one of claims 8, 9, 12 or 13.

15. A method for producing a fermented product, comprising:
(a) optionally performing the method of any one of claims 1 to 7, 10 and 11;
(b) fermenting a wort or a must with the hybrid yeast strain of (a) or the hybrid or derivative yeast strain according to any one of claims 8, 9, 12 or 13;
optionally wherein:
(I) the method further comprises distilling and/or maturing the product produced in step (b),
(II) the wort or must is a whisk(e)y wort and the fermented product is a whisk(e)y, further optionally wherein the wort is a grain whisk(e)y wort or a malt whisk(e)y wort, and/or
(III) the fermentation is at least partially performed at a temperature of 35°C or greater, further optionally wherein the fermentation is at least partially performed at a temperature of 35°C or greater for at least 6 hours.
